# EUROPÄISCHE PATENTSCHRIFT

(11) **EP 2 508 842 B1**
(45) Veröffentlichungstag und Bekanntmachung des Hinweises auf die Patenterteilung: **13.08.2014**
(21) Anmeldenummer: 11002894.1
(22) Anmeldetag: 06.04.2011
(51) Int. Cl.: G01B 9/02, A61B 5/00, G01N 21/47

(54) **Verfahren und System zur optischen Kohärenztomographie**
System and method for optical coherence tomography
Procédé et système destinés à la tomographie de cohérence optique

(43) Veröffentlichungstag der Anmeldung: 10.10.2012
(73) Patentinhaber: Agfa HealthCare N.V., 2640 Mortsel (BE)
(72) Erfinder: Nebosis, Rainer, 81541 München (DE); Reuter, Roland, 83059 Kolbermoor (DE)
(74) Vertreter: Linsmeier, Josef

(56) Entgegenhaltungen:
- US-A- 5 368 033
- US-A1- 2008 151 187
- N. G. STROUTHIDIS ET AL: "A Comparison of Optic Nerve Head Morphology Viewed by Spectral Domain Optical Coherence Tomography and by Serial Histology", INVESTIGATIVE OPHTHALMOLOGY & VISUAL SCIENCE, Bd. 51, Nr. 3, 1. März 2010 (2010-03-01), Seiten 1464-1474, XP55006892, ISSN: 0146-0404, DOI: 10.1167/iovs.09-3984
- TAN O ET AL: "Detection of Macular Ganglion Cell Loss in Glaucoma by Fourier-Domain Optical Coherence Tomography", 1. Dezember 2009 (2009-12-01), OPHTHALMOLOGY, J. B. LIPPINCOTT CO., PHILADELPHIA, PA, US, PAGE(S) 2305 - 2314.E2, XP026788632, ISSN: 0161-6420 [gefunden am 2009-09-10] * Seite 2305, Absatz 1 - Seite 2307, Absatz 3; Abbildungen 1,2 *
- YU L ET AL: "Variable tomographic scanning with wavelength scanning digital interference holography", 15. April 2006 (2006-04-15), OPTICS COMMUNICATIONS, NORTH-HOLLAND PUBLISHING CO. AMSTERDAM, NL, PAGE(S) 462 - 468, XP025252671, ISSN: 0030-4018 [gefunden am 2006-04-15] * Abschnitt "1. Introduction" *

## Beschreibung

Die vorliegende Erfindung betrifft ein Verfahren sowie ein entsprechendes System zur optischen Kohärenztomographie.

Die optische Kohärenztomographie (OCT) ist eine Methode, um lichtstreuende Proben in ihrem Inneren zu vermessen. Biologisches Gewebe ist aufgrund seiner lichtstreuenden Eigenschaften für die diagnostische Untersuchung mittels OCT besonders geeignet. Da die OCT mit relativ geringen Lichtintensitäten auskommt und die Wellenlängen des verwendeten Lichts zumeist in nahen Infrarotbereich (750 nm bis 1350 nm) liegen, stellt sie im Gegensatz zur ionisierenden Röntgendiagnostik für biologisches Gewebe keine Strahlenbelastung dar. Sie ist somit besonders für die Medizin von Bedeutung und grob vergleichbar mit der Ultraschalldiagnostik, wobei anstelle von Schall bei der OCT Licht verwendet wird. Die Laufzeiten des an unterschiedlichen Grenzschichten in der Probe reflektierten Lichts werden mit Hilfe eines Interferometers erfasst. Mit der OCT sind typischerweise um ein bis zwei Größenordnungen höhere Auflösungen als mit Ultraschall zu erreichen, jedoch ist die erzielbare Vermessungstiefe deutlich kleiner. Die gewonnenen Querschnittsbilder reichen aufgrund von optischer Streuung in der Regel nur bis zu einer Tiefe von wenigen Millimetern in das Gewebe hinein. Die derzeit wichtigsten Anwendungsbereiche der OCT liegen in der Ophthalmologie, der Dermatologie sowie der Krebsdiagnose. Es existieren allerdings auch einige nichtmedizinische Anwendungen, wie z.B. in der Werkstoffprüfung.

Insbesondere bei medizinischen Anwendungen der OCT werden an Verfahren und Systeme besondere Anforderungen gestellt, um eine möglichst zuverlässige Untersuchung des Objekts zu gewährleisten.

Aus US 2008/0151187 A1 ist bekannt, auf der Grundlage tomographischer OCT-Bilder (G1 bis Gm) mittels Interpolation jeweils benachbarter tomographischer Bilder (Gi und Gi+1) ein dreidimensionales Bild zu ermitteln.

In N.G. Strouthidis et al., A Comparison of Optic Nerve Head Morphology Viewed by Spectral Domain Optical Coherence Tomography and by Serial Histology, Investigative Ophthalmology & Visual Science, Bd. 51, Nr. 3, März 2010, Seiten 1464-1474, wird ein Verfahren zur Extraktion von interpolierten B-Scans aus einem dreidimensionalen OCT-Volumen beschrieben, bei welchem der Wert eines Pixels im Volumen mit dem kürzesten Schwerpunkt-Schwerpunkt-Abstand ausgewählt wird.

Es ist Aufgabe der vorliegenden Erfindung, ein Verfahren sowie ein entsprechendes System zur optischen Kohärenztomographie anzugeben, welches eine zuverlässige Untersuchung eines Objekts ermöglicht.

Diese Aufgabe wird durch das Verfahren bzw. das System gemäß den unabhängigen Ansprüchen gelöst.

Bei dem erfindungsgemäßen Verfahren zur optischen Kohärenztomographie werden mittels einer optischen Kohärenztomographieeinrichtung mindestens zwei zweidimensionale Ausgangsbilder eines Objekts in voneinander beabstandeten, parallel zueinander verlaufenden Ebenen des Objekts aufgenommen, wobei die Ausgangsbilder jeweils eine Vielzahl von Ausgangsbildwerten, insbesondere Intensitätswerten, umfassen. Ferner wird eine Interpolation der Ausgangsbildwerte der mindestens zwei zweidimensionalen Ausgangs bilder im dreidimensionalen Raum durchgeführt, wobei Interpolationswerte erhalten werden, die ein zweidimensionales Endbild bilden.

Das erfindungsgemäße System zur optischen Kohärenztomographie umfasst eine optische Kohärenztomographieeinrichtung zur Aufnahme von mindestens zwei zweidimensionalen Ausgangsbildern eines Objekts in voneinander beabstandeten, parallel zueinander verlaufenden Ebenen des Objekts, wobei die Ausgangsbilder jeweils eine Vielzahl von Ausgangsbildwerten, insbesondere Intensitätswerten, umfassen, und umfasst eine Verarbeitungseinrichtung zur Interpolation der Ausgangsbildwerte der mindestens zwei zweidimensionalen Ausgangsbilder im dreidimensionalen Raum, wobei Interpolationswerte erhalten werden, die ein zweidimensionales Endbild bilden.

Es liegen dabei jeweils die Ausgangsbildwerte der mindestens zwei zweidimensionalen Ausgangsbilder in einem regelmässigen Gitter im dreidimensionalen Raum, und die Erfindung ist dadurch gekennzeichnet, dass benachbarte Ausgangsbildwerte in dem Gitter in allen drei Raumrichtungen gleiche Abstände aufweisen.

Die Erfindung basiert auf dem Gedanken, mit der optischen Kohärenztomographieeinrichtung zunächst zwei Ausgangsschnittbilder in zwei parallel verlaufenden Ebenen des Objekts aufzunehmen und dann die Ausgangsbildwerte der beiden Ausgangsschnittbilder im dreidimensionalen Raum, welcher durch die beiden voneinander beabstandeten Ausgangsschnittbilder aufgespannt wird, so zu interpolieren, dass ein zweidimensionales Endschnittbild aus Interpolationswerten, d.h. interpolierten Ausgangsbildwerten, erhalten wird. Bei der Ableitung der Interpolationswerte werden hierbei Ausgangsbildwerte nicht nur eines Ausgangsschnittbildes sondern beider Ausgangsschnittbilder interpoliert. Ein Interpolationswert wird hierbei aus mindestens einem Ausgangsbildwert eines ersten Ausgangsschnittbildes und mindestens einem Ausgangsbildwert eines zweiten Ausgangsschnittbildes abgeleitet. Beispielsweise wird ein Interpolationswert aus acht Ausgangsbildwerten abgeleitet, wobei vier Ausgangsbildwerte aus dem ersten Ausgangsschnittbild und vier Ausgangsbildwerte aus dem zweiten Ausgangsschnittbild stammen.

Bei den mit dem erfindungsgemäßen Verfahren bzw. System erhaltenen OCT-Bildern treten Hohlräume oder andere Strukturen mit einer Größe von mehr als etwa 10 µm deutlicher hervor, als dies bei den jeweiligen Ausgangsschnittbildern der Fall ist. Aber auch kleinere Strukturen können zuverlässiger und schneller identifiziert und ggf. untersucht werden. Gerade im Bereich der Dermatologie können dadurch bestimmte diagnostisch relevante bzw. interessante Strukturen in der Haut mit besonders hoher Zuverlässigkeit erkannt und untersucht werden.

Vorzugsweise liegen die Ausgangsbildwerte der mindestens zwei zweidimensionalen Ausgangsbilder in einem regelmäßigen Gitter im dreidimensionalen Raum, wobei benachbarte Ausgangsbildwerte in allen drei Raumrichtungen gleiche Abstände, insbesondere zwischen etwa 2 µm und 4 µm, zueinander aufweisen. Die erfindungsgemäße Interpolation der Ausgangsbildwerte der beiden Ausgangsbilder lässt sich dadurch auf einfache Weise realisieren.

Des Weiteren ist bevorzugt, dass die Ausgangsbildwerte der mindestens zwei zweidimensionalen Ausgangsbilder durch eine trilineare Interpolation und/oder eine trikubische Interpolation interpoliert werden. Bei einer trilinearen bzw. trikubischen Interpolation handelt es sich um ein Verfahren zur Interpolation in einem dreidimensionalen regelmäßigen Gitter, d.h. einem Gitter mit gleicher Gitterkonstante in allen drei Raumrichtungen, wobei ein im Mittelpunkt der jeweiligen Gitterzellen des Gitters liegender Interpolationswert aus jeweils acht Ausgangsbildwerten, die an den acht Ecken der Gitterzelle liegen, durch lineare bzw. kubische Interpolation ermittelt wird. Die an den acht Ecken der Gitterzelle befindlichen Ausgangsbildwerte stellen die nächsten Nachbarn des jeweiligen Interpolationswertes dar, weshalb die trilineare bzw. trikubische Interpolation auch als eine Nächste-Nachbar-Interpolation aufgefasst werden kann. Durch diese Art der Interpolation werden Endbilder mit einem besonders hohen diagnostischen Aussagewert erhalten.

Anstelle oder zusätzlich zu einer linearen bzw. kubischen Interpolation der Ausgangsbildwerte nächster Nachbarn kann auch eine quadratische Interpolation durchgeführt werden. In diesem Fall werden dann die Ausgangsbildwerte der mindestens zwei zweidimensionalen Ausgangsbilder durch eine triquadratische Interpolation interpoliert.

Vorzugsweise sind die mindestens zwei zweidimensionalen Ausgangsbilder des Objekts Echtzeitbilder, welche mit einer Rate von mindestens einem Bild pro Sekunde, vorzugsweise mindestens fünf Bildern pro Sekunde, aufgenommen werden. Dies ist vorteilhafterweise möglich, da die erfindungsgemäße Interpolation ein einfaches und sehr schnelles Verfahren darstellt, das ohne zeitliche Verzögerung auf in Echtzeit aufgenommene OCT-Bilder angewandt werden kann. In Echtzeit aufgenommene Ausgangsbilder können auf diese Weise auch in Echtzeit, d.h. mit einer entsprechenden Bildwiederholungsrate, in Form von erfindungsgemäß interpolierten Endbildern mit entsprechend besser zu erfassenden diagnostischen Informationen auf einer entsprechenden Anzeigeeinrichtung wiedergegeben werden.

Bei einer weiteren vorteilhaften Ausgestaltung der Erfindung werden die mindestens zwei zweidimensionalen Ausgangsbilder des Objekts in voneinander beabstandeten Ebenen des Objekts in einem ersten Betriebsmodus aufgenommen, bei welchem vom Objekt reflektiertes bzw. zurückgestreutes Licht nur von einer Teilfläche, insbesondere von zwei benachbarten Zeilen, eines ortsauflösenden Detektors der optischen Kohärenztomographieeinrichtung erfasst wird, während der optische Abstand eines Reflektors zu einem Strahlteiler der optischen Kohärenztomographieeinrichtung um einen optischen Weg verändert wird, der wesentlich, insbesondere mindestens 100 mal, größer ist als die mittlere Wellenlänge von in die optische Kohärenztomographieeinrichtung eingekoppeltem Licht. Dieser Betriebsmodus erlaubt die Aufnahme von Ausgangsbildern in Form von Tiefenschnitten, sogenannten Slices, mit hoher Geschwindigkeit und folglich in Echtzeit, d.h. mit einer Rate von mindestens einem Bild pro Sekunde, auf einfache Weise und mit hoher Zuverlässigkeit. Die erhöhte diagnostische Aussagekraft der auf diese Weise erfassten und interpolierten Echtzeitbilder erlaubt eine noch zuverlässigere Untersuchung des Objekts.

Bei einer weiteren ebenfalls bevorzugten Ausführung der Erfindung werden die mindestens zwei zweidimensionalen Ausgangsbilder des Objekts in voneinander beabstandeten Ebenen des Objekts in einem zweiten Betriebsmodus aufgenommen werden, bei welchem während einer Veränderung des optischen Abstandes eines Reflektors zu einem Strahlteiler der optischen Kohärenztomographieeinrichtung das von dem Objekt reflektierte Licht von Detektorelementen eines Detektors mehrmals, insbesondere höchstens fünfmal, erfasst wird, wobei die Veränderung des optischen Abstands des Reflektors zum Strahlteiler höchstens das Vierzigfache der mittleren Wellenlänge von in die optische Kohärenztomographieeinrichtung eingekoppeltem Licht beträgt. In diesem Betriebsmodus können Ausgangsbilder in Form von zweidimensionalen Tomogrammen, sogenannten En-face-Bildern, mit hoher Wiederholungsrate, insbesondere in Echtzeit, aufgenommen werden. Auch bei dieser Ausführung ermöglicht die erhöhte diagnostische Aussagekraft der auf diese Weise erfassten und interpolierten Echtzeitbilder eine noch zuverlässigere Untersuchung des Objekts. Vorzugsweise verlaufen hierbei die beiden Ebenen des Objekts in unterschiedlichen Tiefen oberhalb bzw. unterhalb einer mittleren Tiefe im Objekt. Insbesondere verlaufen die beiden Ebenen in einem gleichen Abstand oberhalb bzw. unterhalb der mittleren Tiefe im Objekt. Vorzugsweise entspricht hierbei der Abstand der beiden Ebenen zueinander dem in allen drei Raumrichtungen gleichen Abstand der Ausgangsbildwerte. Durch diese Maßnahmen kann die Ermittlung der Interpolationswerte mit einer besonders hohen Zuverlässigkeit gewährleistet werden.

Bei einer weiteren vorteilhaften Weiterbildung der Erfindung wird die mittlere Tiefe bzw. die unterschiedlichen Tiefen der oberhalb bzw. unterhalb der mittleren Tiefe im Objekt verlaufenden Ebenen durch den Abstand des Reflektors vom Strahlteiler eingestellt. Vorzugsweise wird hierbei der optische Abstand des Reflektors zum Strahlteiler der optischen Kohärenztomographieeinrichtung um einen optischen Weg verändert, der wesentlich, insbesondere mindestens 100 mal, größer ist als die mittlere Wellenlänge des in die optische Kohärenztomographieeinrichtung eingekoppelten Lichts. Die Aufnahme der Ausgangsbilder in Form von En-face-Bildern in unterschiedlichen Ebenen des Objekts kann dadurch auf einfache und schnelle Weise realisiert werden.

Bei einer anderen bevorzugten Ausgestaltung ist vorgesehen, dass vor Durchführung der Interpolation die Anzahl ursprünglicher Ausgangsbildwerte der mindestens zwei zweidimensionalen Ausgangsbilder in zumindest einer Dimension, insbesondere in Richtung der Tiefe des Objekts, reduziert wird, indem jeweils mindestens zwei, vorzugsweise mehr als zehn, ursprüngliche Ausgangsbildwerte zu einem Ausgangsbildwert zusammengefasst werden. Insbesondere handelt es sich bei den ursprünglichen Ausgangsbildwerten um Abtastwerte, die durch sukzessives Abtasten eines aus unterschiedlichen Tiefen innerhalb eines Tiefenbereichs des Objekts erhaltenen Interferenzmuster erhalten werden. Vorzugsweise werden dabei diejenigen ursprünglichen Ausgangsbildwerte zusammengefasst, welche von einem Bereich, insbesondere Tiefenbereich, des Objekts erhalten werden, dessen Ausdehnung in der mindestens einen Dimension der Auflösung, insbesondere der axialen Auflösung oder Tiefenauflösung, der optischen Kohärenztomographieeinrichtung entspricht.

Durch diese Maßnahmen wird einerseits berücksichtigt, dass die Abtastung des Interferenzsignals hoch genug sein muss, um das sogenannte Abtasttheorem nicht zu verletzen. Andererseits wird dadurch auch der Tatsache Rechnung getragen, dass der Abstand zweier Abtastwerte des Interferenzsignals in der Regel deutlich kleiner ist als die physikalische Auflösung der abbildenden Optik der optischen Kohärenztomographieeinrichtung. Dies bedeutet, dass mehrere, vorzugsweise mehr als zehn, aufeinander folgende Abtastwerte annähernd dieselbe physikalische Information enthalten und daher ohne signifikanten Informationsverlust zu einem Wert zusammengefasst werden können. Der Ausgangsbildwert entspricht dabei beispielsweise einem Mittelwert oder dem Median aus den ursprünglichen Ausgangsbildwerten. Dadurch wird die erfasste Datenmenge in Form der ursprünglichen Ausgangsbildwerte ohne signifikante Informationsverluste erheblich reduziert und darüber hinaus die für eine besonders zuverlässige Durchführung der Interpolation erforderlichen gleichen Abstände der Ausgangsbildwerte in allen drei Raumrichtungen leicht realisierbar.

Es ist außerdem bevorzugt, dass die Schärfentiefe der abbildenden Optik der optischen Kohärenztomographieeinrichtung größer ist als der, vorzugsweise in allen drei Raumrichtungen identische, räumliche Abstand der Ausgangsbildwerte voneinander. Dadurch werden die Ausgangsbilder und die daraus durch Interpolation gewonnen Endbilder stets mit der erforderlichen Genauigkeit erfasst bzw. gewonnen.

Weitere Vorteile, Merkmale und Anwendungsmöglichkeiten der vorliegenden Erfindung ergeben sich aus der nachfolgenden Beschreibung in Zusammenhang mit den Figuren. Es zeigen:
- Fig. 1: eine schematische Darstellung eines Beispiels einer optischen Kohärenztomographieeinrichtung;
- Fig. 2: eine schematische Darstellung eines Beispiels einer Detektorfläche zur Veranschaulichung eines ersten Betriebsmodus;
- Fig. 3: ein Raumelement des Objekts mit Schnitten in ersten Ebenen zur Veranschaulichung des ersten Betriebsmodus;
- Fig. 4: ein Raumelement des Objekts mit einem Schnitt in einer zweiten Ebene zur Veranschaulichung eines zweiten Betriebsmodus;
- Fig. 5: ein Raumelement des Objekts mit Schnitten in zweiten Ebenen zur Veranschaulichung eines dritten Betriebsmodus;
- Fig. 6: a) und b) zwei Querschnitte durch das Objekt und den Probenarm des Interferometers zur Veranschaulichung der Fokusnachführung;
- Fig. 7: ein Beispiel für ein regelmäßiges Gitter zur Veranschaulichung der Interpolation von Ausgangsbildwerten;
- Fig. 8: ein Schema zur Veranschaulichung einer Abtastung eines Interferenzmusters in Richtung der Tiefe eines Objekts im Vergleich zur physikalischen Auflösung in Richtung der Tiefe;
- Fig. 9: ein weiteres Schema zur Veranschaulichung einer Zusammenfassung von in Richtung der Tiefe eines Objekts abgetasteten ursprünglichen Ausgangsbildwerten zu jeweils einem Ausgangsbildwert im Vergleich zur physikalischen Auflösung in Richtung der Tiefe;
- Fig. 10: ein weiteres Schema zur Veranschaulichung der Interpolation der Ausgangsbildwerte von zwei in Richtung der Tiefe des Objekts erhaltenen Ausgangsbildern;
- Fig. 11: ein weiteres Schema zur Veranschaulichung der Erfassung Ausgangsbildwerte in einer (links) bzw. zwei (rechts) Ebenen quer zur Richtung der Tiefe eines Objekts sowie der Interpolation der Ausgangsbildwerte der von den beiden Ebenen erhaltenen Ausgangsbildern (rechts);
- Fig. 12: ein Beispiel eines Ausgangsbildes (links) im Vergleich zu einem entsprechenden Endbild (rechts), welches durch die beschriebene Interpolation erhalten wurde;
- Fig. 13: eine schematische Darstellung eines Systems zur Durchführung des erfindungsgemäßen Verfahrens zur optischen Kohärenztomographie;
- Fig. 14: eine Darstellung eines Messkopfes des Systems;
- Fig. 15: eine Bildschirmansicht zur Veranschaulichung der Eingabe von Patientendaten;
- Fig. 16: eine Bildschirmansicht zur Veranschaulichung der Anzeige der eingegebenen Patientendaten;
- Fig. 17: eine Bildschirmansicht zur Veranschaulichung der Einstellung der Hautfeuchtigkeit;
- Fig. 18: eine weitere Bildschirmansicht zur Veranschaulichung der Einstellung der Hautfeuchtigkeit;
- Fig. 19: eine erste Bildschirmansicht zur Veranschaulichung der Wahl einer zweiten Ebene anhand eines Slice-Bildes;
- Fig. 20: eine zweite Bildschirmansicht zur Veranschaulichung der Wahl der zweiten Ebene anhand des Slice-Bildes;
- Fig. 21: eine dritte Bildschirmansicht zur Veranschaulichung der Wahl der zweiten Ebene anhand des Slice-Bildes;
- Fig. 22: eine vierte Bildschirmansicht zur Veranschaulichung der Wahl der zweiten Ebene anhand des Slice-Bildes;
- Fig. 23: eine fünfte Bildschirmansicht zur Veranschaulichung der Wahl der zweiten Ebene anhand des Slice-Bildes;
- Fig. 24: eine sechste Bildschirmansicht zur Veranschaulichung der Anzeige eines auf einen Benutzerbefehl hin gespeicherten Slice-Bildes sowie der Wahl der zweiten Ebene anhand eines temporär gespeicherten Slice-Bildes;
- Fig. 25: eine Bildschirmansicht zur Veranschaulichung der Wahl einer ersten Ebene für ein aufzunehmendes Slice-Bild anhand eines En-face-Bildes;
- Fig. 26: eine Bildschirmansicht zur Veranschaulichung der Wahl von Slice- sowie En-face-Bildern, die einem dreidimensionalen Tomogramm entstammen, in einem Bildbetrachtungsmodus;
- Fig. 27: eine Bildschirmansicht zur Veranschaulichung einer Eingabe von Kommentaren im Bildbetrachtungsmodus;
- Fig. 28: eine Bildschirmansicht zur Veranschaulichung eines Verwaltungsmodus des Systems; und
- Fig. 29: ein Beispiel für einen automatisch erstellten Untersuchungsbericht.

### 1. Optische Kohärenztomographieeinrichtung

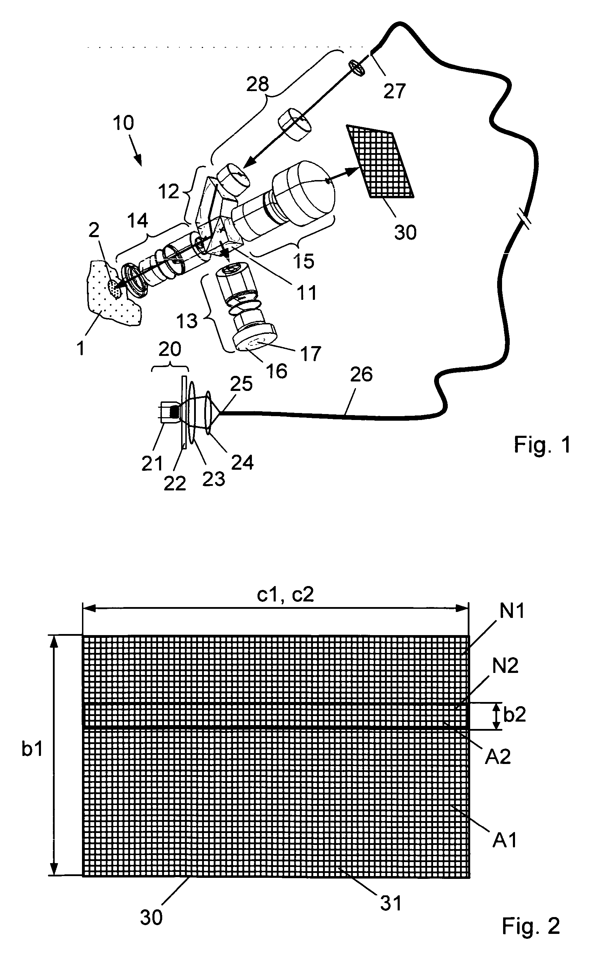

Figur 1 zeigt eine schematische Darstellung eines Beispiels einer optischen Kohärenztomographieeinrichtung, nachfolgend auch als OCT-Einrichtung bezeichnet, mit einem Interferometer 10, welches einen Strahlteiler 11, einen Beleuchtungsarm 12, einen Referenzarm 13, einen Probenarm 14 sowie einen Detektorarm 15 umfasst. Ferner ist eine Strahlungsquelle 21 zur Erzeugung von Licht vorgesehen, welches durch einen optischen Filter 22 gefiltert wird und durch eine aus Linsen 23 und 24 zusammengesetzte Optik auf einen Eingangsbereich 25 eines Lichtleiters 26 fokussiert wird. Die Strahlungsquelle 21 bildet zusammen mit dem optischen Filter 22 eine Einrichtung, welche auch als Lichtquelle 20 bezeichnet wird.

Das in den Lichtleiter 26 eingekoppelte Licht wird durch eine in dessen Ausgangsbereich 27 befindliche Optik 28 in den Beleuchtungsarm 12 des Interferometers 10 eingekoppelt. Von dort gelangt das Licht zunächst zum Strahlteiler 11, durch welchen dieses einerseits in den Referenzarm 13 weitergeleitet und von einem an dessen Ende befindlichen beweglichen Referenzspiegel 16 reflektiert wird und andererseits nach Durchlaufen des Probenarms 14 eine Fläche 2 einer Probe 1 beleuchtet.

Das von der Probe 1 reflektierte, insbesondere zurückgestreute, Licht durchläuft erneut den Probenarm 14, wird im Strahlteiler 11 mit dem am Referenzspiegel 16 reflektierten Licht aus dem Referenzarm 13 überlagert und gelangt schließlich über den Detektorarm 15 auf einen Detektor 30, der eine Vielzahl von in einer, vorzugsweise ebenen, Fläche angeordneten Detektorelemente umfasst und folglich eine ortsaufgelöste Erfassung des von der Probe 30 reflektierten Lichts bzw. eines entsprechenden Interferenzmusters aufgrund dessen Überlagerung mit dem am Referenzspiegel 16 reflektierten Licht ermöglicht.

Als Detektor 30 wird vorzugsweise eine CMOS-Kamera eingesetzt, deren Detektorelemente (sogenannte Pixel) im infraroten Spektralbereich empfindlich sind, insbesondere in einem Spektralbereich zwischen etwa 1250 nm und 1350 nm. Vorzugsweise hat die CMOS-Kamera 512 x 640 Detektorelemente.

Als Lichtleiter 26 dient vorzugsweise eine sogenannte Multimode-Faser, deren numerische Apertur und Kerndurchmesser es zulassen, dass sich bei einer bestimmten Wellenlänge des in die Faser eingekoppelten Lichts nicht nur eine Fasermode ausbilden kann, sondern viele verschiedene Fasermoden angeregt werden können. Vorzugsweise beträgt der Durchmesser der verwendeten Multimode-Faser zwischen etwa 1 mm und 3 mm, insbesondere etwa 1,5 mm.

Die Größe der beleuchteten Fläche 2 auf der Probe 1 entspricht in etwa der Größe der beleuchteten Fläche 17 auf dem Referenzspiegel 16 und wird einerseits durch die am Eingangsbereich des Lichtleiters 26 befindliche Optik, welche im dargestellten Beispiel die Linsen 23 und 24 umfasst, und andererseits durch die im Ausgangsbereich des Lichtleiters 26 vorgesehene Optik 28 bestimmt.

Bei der beschriebenen OCT-Einrichtung wird das entstehende Interferenzmuster mit dem Detektor 30 erfasst, wobei ein entsprechendes Interferenzsignal erzeugt wird. Die Abtastrate des Detektors 30 zum Abtasten des Interferenzsignals muss dabei so gewählt werden, dass die zeitliche Variation des Interferenzmusters mit ausreichender Genauigkeit erfasst werden kann. Dies erfordert im Allgemeinen hohe Abtastraten, wenn hohe Geschwindigkeiten für einen Tiefenscan erzielt werden sollen.

Ein Tiefenscan wird bei dem beschriebenen System vorzugsweise dadurch realisiert, dass der optische Abstand des Referenzspiegels 16 vom Strahlteiler 11 während der Erfassung des von der Probe 1 reflektierten Lichts mit dem Detektor 30 mit einer Geschwindigkeit v um einen optischen Weg verändert wird, welcher wesentlich größer ist als die mittlere Wellenlänge des in das Interferometer 10 eingekoppelten Lichts. Vorzugsweise wird hierbei das in mindestens 100 unterschiedlichen Tiefen der Probe 1 reflektierte Licht vom Detektor 30 erfasst. Es ist insbesondere bevorzugt, dass der optische Weg periodisch mit einer Amplitude verändert wird, die wesentlich größer ist als die mittlere Wellenlänge des in das Interferometer 10 eingekoppelten Lichts. Die Änderung des optischen Abstands des Referenzspiegels 16 um dem optischen Weg bzw. mit der Amplitude ist vorzugsweise mindestens 100 mal, insbesondere mindestens 1000 mal, größer ist als die mittlere Wellenlänge des in das Interferometer 10 eingekoppelten Lichts. Aufgrund der großen Wege bei dieser Abstandsvariation wird diese Bewegung des Referenzspiegels 16 auch als makroskopische Bewegung bezeichnet.

Da die einzelnen Perioden eines Interferenzmusters im Allgemeinen jeweils zu mehreren Zeitpunkten abgetastet werden müssen, ist die maximal mögliche Scan-Geschwindigkeit in Richtung der Tiefe der Probe 1 abhängig von der maximal möglichen Abtastrate des Detektors 30. Bei Verwendung von schnellen Detektor-Arrays mit hoher räumlicher Auflösung, d.h. großer Anzahl von Detektorelementen pro Längeneinheit, liegt die maximale Abtastrate typischerweise im Bereich von etwa 1 kHz. Dies führt bei einer mittleren Wellenlänge des in das Interferometer eingekoppelten Lichts von beispielsweise 1300 nm zu einer maximalen Geschwindigkeit für den Tiefenscan von etwa 0,1 mm/s, wenn vier Punkte pro Periode einer Interferenzstruktur aufgenommen werden.

Zur Erhöhung der Geschwindigkeit des Tiefenscans wird in der vorliegenden OCT-Einrichtung der zeitliche Verlauf der Empfindlichkeit des Detektors 30 für das zu erfassende Licht mit einer Frequenz moduliert, die um bis zu 40 % größer oder kleiner sein kann als die Dopplerfrequenz f_{D}, wobei die Dopplerfrequenz f_{D} durch die mittlere Wellenlänge λ₀ des in das Interferometer 10 eingekoppelten Lichts und die Geschwindigkeit v des beweglichen Referenzspiegels 16 wie folgt gegeben ist: f_{D} = 2v/λ₀ Typische Frequenzen dieser Modulation liegen im Bereich zwischen 1 kHz und 25 kHz. Es ist besonders bevorzugt, dass die Frequenz der Modulation der Detektorempfindlichkeit ungleich der Dopplerfrequenz f_{D} ist.

Das von der Probe 1 reflektierte und auf den Detektor 30 treffende Licht überlagert sich hierbei mit der modulierten Empfindlichkeit des Detektors 30, so dass der Detektor 30 bei der Erfassung des auf den Detektor 30 treffenden Interferenzmusters anstelle eines hochfrequenten Interferenzsignals mit einer Vielzahl von Perioden ein niederfrequentes Schwebungssignal erzeugt, welches deutlich weniger Perioden aufweist als das hochfrequente Interferenzsignal. Bei der Abtastung dieser Schwebung sind daher deutlich weniger Abtastzeitpunkte pro Zeiteinheit erforderlich, ohne hierbei relevante Information zu verlieren, als bei einer Abtastung des hochfrequenten Interferenzsignals ohne die Modulation der Empfindlichkeit des Detektors 30. Bei einer gegebenen maximalen Abtastrate des Detektors 30 hat dies zur Folge, dass die maximale Geschwindigkeit für einen Tiefenscan des Systems um ein Vielfaches erhöht werden kann.

Die Empfindlichkeit des Detektors 30 lässt sich z.B. direkt oder mit einem vor dem Detektor 30 angeordneten steuerbaren elektronischen Shutter modulieren. Alternativ oder zusätzlich können Eigenschaften eines optischen Elements vor dem Detektor 30, wie z.B. die Durchlässigkeit eines Detektorobjektivs für das von der Probe 1 reflektierte Licht, moduliert werden. Im Vergleich zu Systemen mit einer konstanten Detektorempfindlichkeit wird hierdurch die Scan-Geschwindigkeit um den Faktor 4 oder sogar 8 erhöht.

Die Geschwindigkeit der Bewegung des Referenzspiegels 16 steht vorzugsweise in einer festen Beziehung zur Frequenz der Modulation der Empfindlichkeit des Detektors 30 und ist insbesondere so gewählt, dass in eine Periodendauer des entstehenden Schwebungssignals eine ganzzahlige Anzahl von Abtastzeitpunkten, vorzugsweise vier Abtastzeitpunkte, passen.

Die auf diese Weise abgetasteten Schwebungssignale müssen vor einer Visualisierung noch verarbeitet werden, da in diesen Signalen noch die Interferenzinformation enthalten ist. Die wesentliche Information, die visualisiert werden soll, ist die Amplitude und Tiefenposition der jeweiligen Interferenz, nicht jedoch die Interferenzstruktur selbst. Dazu muss das Schwebungssignal demoduliert werden, indem z.B. durch Fourier- oder Hilpert-Transformation die sog. Einhüllende des Schwebungssignals bestimmt wird.

Da die Phase des Schwebungssignals im Allgemeinen unbekannt ist und diese sich auch für unterschiedliche Schwebungssignale aus unterschiedlichen Tiefen unterscheiden kann, wird ein digitaler Demodulationsalgorithmus eingesetzt, der unabhängig von der Phase ist. Vorzugsweise werden für die Abtastung des Interferenzsignals mit vier Abtastzeitpunkten pro Periode sogenannte 90° Phase Shift-Algorithmen verwendet. Hierdurch wird eine schnelle Demodulation des Schwebungssignals erreicht.

Vorzugsweise umfasst eine Periode der Modulation der Empfindlichkeit des Detektors 30 zwei Teilperioden, wobei während einer ersten Teilperiode der Detektor empfindlich und während einer zweiten Teilperiode der Detektor unempfindlich ist für das zu erfassende Licht. Im Allgemeinen sind die erste und die zweite Teilperiode gleich lang. Es kann jedoch von Vorteil sein, die Dauer der ersten und zweiten Teilperiode unterschiedlich lang zu gestalten. Dies gilt beispielsweise dann, wenn die Intensität des von der Lichtquelle 20 abgegebenen bzw. in das Interferometer 10 eingekoppelten Lichts und/oder des von der Probe 1 reflektierten Lichts relativ gering ist. In diesen Fällen kann die erste Teilperiode so gewählt werden, dass deren Dauer länger ist als die Dauer der zweiten Teilperiode. Auf diese Weise wird auch bei geringen Lichtintensitäten neben einer hohen Tiefenscan-Geschwindigkeit ein hohes Signal-Rausch-Verhältnis und damit eine hohe Bildqualität gewährleistet.

Alternativ zur Empfindlichkeit des Detektors 30 kann auch die Intensität des in das Interferometer 10 eingekoppelten Lichts zeitlich moduliert werden, wobei hinsichtlich der bevorzugten Ausführungen sowie der vorteilhaften Wirkungen die Erläuterungen zur oben beschriebenen Modulation der Detektorempfindlichkeit entsprechend gelten.

Die Strahlungsquelle 21 umfasst vorzugsweise einen wendelförmigen Draht, welcher von einer transparenten Umhüllung, vorzugsweise aus Glas, umgeben ist. Vorzugsweise ist die Strahlungsquelle 21 als Halogenglühlampe, insbesondere Wolfram-Halogen-Glühlampe, ausgebildet, wobei als Draht ein Wolframdraht verwendet wird und das Innere der Umhüllung mit Gas gefüllt ist, welches ein Halogen, beispielsweise Jod oder Brom, enthält. Durch Anlegen einer elektrischen Spannung wird der wendelförmige Draht zum Glühen gebracht, wodurch dieser räumlich inkohärentes Licht aussendet. Unter räumlich inkohärentem Licht im Sinne der vorliegenden Erfindung ist Licht zu verstehen, dessen räumliche Kohärenzlänge kleiner ist als 15 µm und insbesondere nur einige wenige Mikrometer, d.h. zwischen etwa 1 µm und 5 µm, beträgt.

Das von der Strahlungsquelle 21 erzeugte räumlich inkohärente Licht passiert den optischen Filter 22, welcher als Bandpassfilter ausgebildet ist und im Wesentlichen nur für Licht innerhalb einer vorgebbaren spektralen Bandbreite durchlässig ist. Der optische Filter 22 weist eine glockenförmige oder gaußförmige spektrale Filtercharakteristik auf, wobei nur diejenigen spektralen Lichtanteile des von der Strahlungsquelle 21 erzeugten Lichts den optischen Filter 22 passieren können, welche innerhalb der vorgegebenen Bandbreite um eine mittlere Wellenlänge der glocken- bzw. gaußförmigen spektralen Filtercharakteristik liegen.

Unter einer gaußförmigen spektralen Filtercharakteristik im Sinne der Erfindung ist zu verstehen, dass die Durchlässigkeit des optischen Filters 22 für Licht mit bestimmten Wellenlängen λ proportional ist zu exp[-[(λ - λ₀)/2·Δλ]²], wobei λ₀ die Wellenlänge angibt, bei welcher der optische Filter 22 seine maximale Durchlässigkeit aufweist, und Δλ die Standardabweichung bezeichnet, welche mit der Halbwertsbreite FWHM des gaußförmigen Durchlässigkeitsverlaufs wie folgt zusammenhängt: FWHM ≈ 2,35 · Δλ.

Unter einer glockenförmigen spektralen Filtercharakteristik ist ein spektraler Verlauf der Durchlässigkeit des optischen Filters 22 zu verstehen, welcher durch einen gaußförmigen Verlauf angenähert werden kann und/oder nur soweit von einem gaußförmigen Verlauf abweicht, dass dessen Fourier-Transformierte einen im Wesentlichen gaußförmigen Verlauf mit keinen Nebenmaxima oder nur wenigen, sehr niedrigen Nebenmaxima aufweist, deren Höhe maximal 5 % des Maximums der Fourier-Transformierten beträgt.

Durch die Verwendung einer Strahlungsquelle 21, welche *a priori* räumlich inkohärentes Licht erzeugt, wird bei der Erfassung des von der Probe 1 reflektierten Lichts mittels des zweidimensionalen ortsauflösenden Detektors 30 das Auftreten von sog. Geisterbildern aufgrund kohärenten Übersprechens von Lichtstrahlen aus unterschiedlichen Orten innerhalb der untersuchten Probe 1 vermieden. Auf die bei einer Verwendung von räumlich kohärenten Strahlungsquellen üblicherweise erforderlichen zusätzlichen Einrichtungen zur Zerstörung der räumlichen Kohärenz kann dadurch verzichtet werden.

Darüber hinaus kann hierdurch auf thermische Strahlungsquellen, wie z.B. Glüh- oder Halogenlampen, zur Erzeugung inkohärenten Lichts zurückgegriffen werden, die deutlich leistungsstärker und kostengünstiger sind als die häufig eingesetzten Superlumineszenzdioden (SLDs).

Durch die optische Filterung mit einer gauß- oder glockenförmigen Filtercharakteristik wird das von der Strahlungsquelle 21 erzeugte Licht in zeitlich teilkohärentes Licht mit einer zeitlichen Kohärenzlänge von vorzugsweise mehr als etwa 6 µm umgewandelt. Dies ist bei der beschriebenen OCT-Einrichtung vom Typ eines sog. Time-Domain-OCT, bei welchem die Länge eines Referenzarms 13 im Interferometer 10 verändert und mittels eines, vorzugsweise zweidimensionalen, Detektors 30 kontinuierlich die Intensität der auftretenden Interferenz erfasst wird, besonders vorteilhaft, da durch die Filterung des Lichts mittels des durch den optischen Filter 22 realisierten Bandpasses einerseits eine hohe laterale Auflösung des von der Probe 1 erfassten Bildes erreicht und durch die gauß- oder glockenförmige spektrale Filtercharakteristik des optischen Filters 22 andererseits ein Auftreten von störenden Nebenmaxima bei der FourierTransformation des mit dem Detektor erfassten Interferenzmusters, welche das Auftreten weiterer Geisterbilder verursachen würden, vermieden wird.

Insgesamt werden mit der beschriebenen OCT-Einrichtung auf einfache Weise OCT-Bilder mit hoher Auflösung und Bildqualität erhalten.

Im gezeigten Beispiel ist der optische Filter 22 zwischen der Strahlungsquelle 21 und der aus den beiden Linsen 23 und 24 gebildeten eingangsseitigen Optik angeordnet. Grundsätzlich ist es aber auch möglich, den optischen Filter 22 zwischen den beiden Linsen 23 und 24 oder aber zwischen der Linse 24 und dem Eingangsbereich 25 des Lichtleiters 26 vorzusehen. Grundsätzlich ist eine Anordnung des optischen Filters 22 besonders vorteilhaft, wenn die auf den optischen Filter 22 treffenden Lichtstrahlen nur eine geringe Divergenz aufweisen oder insbesondere parallel zueinander verlaufen, da hierdurch einerseits Reflexionsverluste an den Grenzflächen des optischen Filters 22 reduziert und andererseits ein Strahlversatz aufgrund von Lichtbrechung vermindert wird. Im gezeigten Beispiel ist daher eine Anordnung des optischen Filters 22 zwischen den beiden Linsen 23 und 24 der Optik besonders bevorzugt.

Alternativ oder zusätzlich ist es aber auch möglich, den optischen Filter 22 direkt auf die Umhüllung der Strahlungsquelle 21 aufzubringen. Dies hat den Vorteil, dass auf ein zusätzliches Filterbauteil verzichtet werden kann.

Alternativ oder zusätzlich ist es aber auch möglich, den optischen Filter 22 zwischen dem Ausgangsbereich 27 des Lichtleiters 26 und dem Beleuchtungsarm 12 anzuordnen, beispielsweise vor oder zwischen den Linsen der zwischen dem Ausgangsbereich 27 des Lichtleiters 26 und dem Eingang des Beleuchtungsarms 12 befindlichen Optik 28.

Bei einer einfachen und besonders zuverlässigen Variante umfasst der optische Filter 22 einen Absorptionsfilter, insbesondere ein sogenanntes Masseglas, und einen Interferenzfilter, wobei auf das Masseglas mehrere, vorzugsweise zwischen etwa 30 und 70, dünne Schichten mit unterschiedlichen Brechungsindizes, beispielsweise durch Aufdampfen, aufgebracht werden, wodurch ein Interferenzfilter erhalten wird.

Für den Fall, dass der optische Filter 22 in die Umhüllung der Strahlungsquelle 21 integriert wird, wird der optische Filter 22 vorzugsweise durch Aufbringen solcher Interferenzschichten auf die Umhüllung realisiert. Alternativ oder zusätzlich ist es aber auch möglich, eine oder mehrere der Linsen 23, 24 bzw. der Linsen der Optik 28 mit einem entsprechenden Interferenzfilter zu versehen.

### 2. Betriebsmodi der OCT-Einrichtung

Die beschriebene OCT-Einrichtung kann in drei unterschiedlichen Betriebsmodi betrieben werden. Bei den Betriebsmodi handelt es sich um zwei Echtzeitmodi, in denen OCT-Bilder der Probe 1 mit einer hohen Rate von mindestens einem Bild pro Sekunde, vorzugsweise etwa 5 bis 10 Bildern pro Sekunde, erzeugt werden, sowie einen statischen Betriebsmodus.

Im ersten Betriebsmodus, dem Echtzeitmodus 1, werden in Echtzeit zweidimensionale Tiefenschnitte der Probe 1 erzeugt (sog. Slices). Dies wird dadurch realisiert, dass als Detektor 30 eine CMOS-Kamera verwendet wird, welche die Einstellung eines sog. Window of Interest (WOI) zulässt, bei welcher lediglich eine Teilfläche des Detektors 30 für Licht sensitiv ist und dieses in entsprechende Detektorsignale umwandelt. Die Reduzierung der sensitiven Kamerafläche ist verbunden mit einer deutlichen Erhöhung der Kamerageschwindigkeit, so dass bei dieser Einstellung mehr Kamerabilder pro Sekunde erzeugt werden können als im Vollbild-Modus.

Im Echtzeitmodus 1 wird vorzugsweise ein WOI gewählt, das in einer Richtung der gesamten Kameralänge bzw. -breite entspricht (z.B. 640 Pixel) und in der anderen Richtung die - durch den Typ der jeweiligen Kamera gegebene - minimal mögliche Anzahl von Pixel aufweist (z.B. 4 Pixel). Dadurch wird die Geschwindigkeit der Kamera so weit erhöht, dass OCT-Bilder in Echtzeit aufgenommen werden können.

Dies wird vorzugsweise in Kombination mit der oben beschriebenen Modulation der Empfindlichkeit des Detektors 30 bzw. der Modulation der Intensität des in das Interferometer 10 eingekoppelten Lichts bzw. vom Interferometer 10 ausgegebenen Lichts erreicht.

Figur 2 zeigt als Beispiel einen Detektor 30 mit einer Detektorfläche A1, welche eine erste Anzahl N1 von in einer Ebene angeordneten Detektorelementen 31 umfasst und eine Länge c1 und eine Breite b1 aufweist. Bei der o.g. Einstellung eines WOI wird Licht lediglich von den in einer Teilfläche A2 der Detektorfläche A1 befindlichen Detektorelementen 31 erfasst und in entsprechende Detektorsignale umgewandelt. Die zweite Anzahl N2 der Detektorelemente 31 der Teilfläche A2 ist kleiner als die erste Anzahl N1 der Detektorelemente 31 der gesamten Detektorfläche A1. Die Längen c1 und c2 der Detektorfläche A1 bzw. Teilfläche A2 sind gleich groß, während die Breiten b1 und b2 der Detektorfläche A1 bzw. Teilfläche A2 unterschiedlich sind.

Im gezeigten Beispiel ist die Teilfläche A2 nur vier Pixel breit, wohingegen die Detektorfläche A1 512 Pixel breit ist. Die sensitive Fläche der Detektorfläche A1 wird also um einen Faktor von 128 reduziert, was die für die Erfassung von Interferenzmustern und deren Umwandlung in entsprechende Detektorsignale erforderliche Zeitdauer erheblich verkürzt.

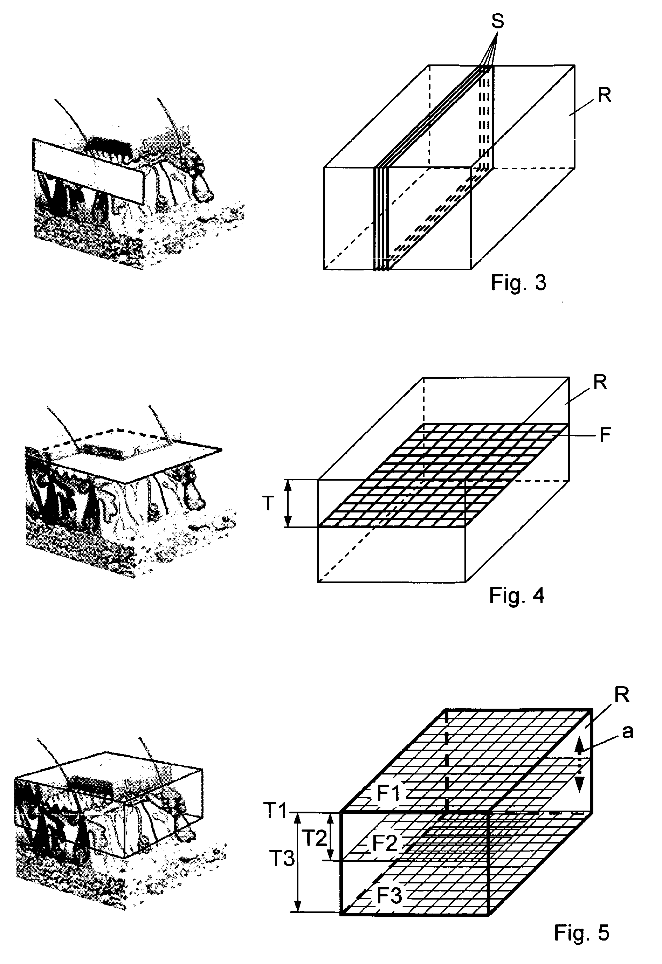

Wie in Figur 3 dargestellt ist, werden in diesem Beispiel anstelle eines vollen dreidimensionalen Tomogramms nur vier (entsprechend den vier Pixelreihen der Teilfläche A2) zweidimensionale Tiefenschnitte S (sogenannte Slices) aus dem betrachteten Raumelement R der Probe 1 erhalten. Aufgrund der im ersten Betriebsmodus erhaltenen Slices wird dieser Modus auch als Slice-Modus bezeichnet.

Zur weiteren Veranschaulichung zeigt der linke Teil der Figur 3 ein Modell der menschlichen Haut, in welches beispielhaft eine Ebene eines im Betriebsmodus 1, vorzugsweise in Echtzeit, aufgenommenen zweidimensionalen Tiefenschnittes bzw. Slices eingezeichnet ist.

Im zweiten Betriebsmodus, dem Echtzeitmodus 2, werden - wie in Figur 4 dargestellt - zweidimensionale Tomogramme F aus einer bestimmten Tiefe T des betrachteten Raumelements R der Probe 1 erzeugt, wobei die Tiefe T frei wählbar ist. Hierbei wird die gesamte Detektorfläche A1 des Detektors 30 für die Erfassung des von der Probe 1 reflektierten Lichts und dessen Umwandlung in entsprechende Detektorsignale genutzt, wobei jedoch nur jeweils maximal fünf Kamerabilder zur Berechnung eines Tomogramms F herangezogen werden. Dazu wird der Referenzspiegel 16 im Interferometer 10 bei einem bestimmten Abstand zum Strahlteiler 11 mit einer Amplitude von etwa 1 µm periodisch um diesen Abstand herum bewegt, während bis zu fünf Kamerabilder aufgenommen werden, die dann zu einem OCT-Bild verrechnet werden. Auf diese Weise können Tomogramme F mit hoher Wiederholungsrate, insbesondere in Echtzeit, erzeugt werden. Im Verhältnis zur oben beschriebenen makroskopischen Bewegung des Referenzspiegels 16 handelt es sich hierbei um eine mikroskopische Bewegung des Referenzspiegels 16.

Durch die makroskopische Bewegung des Referenzspiegels 16 - ggf. in Kombination mit einer weiter unten näher beschriebenen Fokusnachführung des in einer bestimmten Tiefe T in der Probe mittels der im Probenarm 14 befindlichen Probenoptik fokussierten Lichts - kann die Tiefe T, aus der das Tomogramm F gewonnen wird, frei gewählt werden.

Aufgrund der im zweiten Betriebmodus erhaltenen zweidimensionalen Schnitte F durch die Probe 1 in Ebenen, die im Wesentlichen senkrecht zur Richtung des auf die Probe 1 treffenden Lichts verlaufen, wird der zweite Betriebsmodus auch als En-face-Modus bezeichnet.

Zur weiteren Veranschaulichung zeigt der linke Teil der Figur 4 ein Modell der menschlichen Haut, in welches beispielhaft eine Ebene eines im Betriebsmodus 2, vorzugsweise in Echtzeit, aufgenommenen zweidimensionalen Tomogramms bzw. En-face-Bildes eingezeichnet ist.

Im dritten Betriebsmodus, einem statischen Modus, wird ein vollständiger dreidimensionaler Datensatz mit Hilfe der makroskopischen Bewegung des Referenzspiegels 16 in Kombination mit einer Fokusnachführung aufgenommen.

Bei einer mittleren Wellenlänge des in das Interferometer 10 eingekoppelten Lichts im Bereich von beispielsweise 1 µm liegt die optische Weglänge bzw. Amplitude der makroskopischen Bewegung des Referenzspiegels 16 mindestens bei etwa 0,1 mm, vorzugsweise mindestens bei etwa 1 mm.

Im Gegensatz zu der üblichen mikroskopischen Amplitude der Referenzspiegelbewegung in der Größenordnung von Bruchteilen der mittleren Wellenlänge des eingekoppelten Lichts, also von bis zu typischerweise 1 µm, erfolgt bei der beschriebenen OCT-Einrichtung eine makroskopische Bewegung des Referenzspiegels 16 in der Größenordnung von 0,1 mm bis zu mehreren Millimetern. Während der makroskopischen Linearbewegung des Referenzspiegels 16 wird das von der Probe 1 reflektierte Licht über das Interferometer 10 zum zweidimensionalen Detektor 30 weitergeleitet und von diesem sukzessive zu mehreren Zeitpunkten für jeweils eine bestimmte Zeitdauer, welche der Integrationszeit des Detektors 30 entspricht, erfasst und in entsprechende Detektorsignale umgewandelt.

Damit eine Interferenz zwischen dem vom Referenzspiegel 16 und dem von der Probe 1 reflektierten Licht auftreten kann, muss die sog. Kohärenzbedingung erfüllt sein, welche u.a. besagt, dass die jeweils reflektierten Lichtwellen eine konstante Phasenbeziehung zueinander haben müssen, um miteinander interferieren zu können. Aufgrund der Verwendung von Licht mit einer sehr kurzen Kohärenzlänge von typischerweise 10 µm oder weniger ist die Bedingung einer konstanten Phasenbeziehung nur in bestimmten Tiefen oder Tiefenbereichen der Probe 1 erfüllt, welche auch als Kohärenz-Gate bezeichnet werden.

Jede Position des Referenzspiegels 16 während der makroskopischen Bewegung entspricht dabei einer bestimmten Tiefe innerhalb der Probe 1 oder einem Tiefenbereich um diese bestimmte Tiefe herum, für welche bzw. welchen die Kohärenzbedingung erfüllt ist, so dass eine Interferenz zwischen dem vom Referenzspiegel 16 und dem von der Probe 1 reflektierten Licht auftreten kann.

Im Falle einer periodischen Bewegung des Referenzspiegels 16 können beide Halbperioden der periodischen Bewegung des Referenzspiegels 16 jeweils zur Aufnahme von Detektorsignalen genutzt werden.

Auf diese Weise werden durch den Detektor 30 sukzessive zweidimensionale Schnitte aus unterschiedlichen Tiefen der Probe 1 aufgenommen. Dies ist in Figur 5 veranschaulicht, in welcher - stellvertretend für eine Vielzahl von zweidimensionalen Schnitten - ein erster, zweiter und dritter zweidimensionaler Schnitt F1, F2 bzw. F3 durch ein Raumelement R der Probe 1 dargestellt ist. Ein solcher zweidimensionaler Schnitt "wandert" synchron mit der makroskopischen Bewegung des Referenzspiegels 16 in Richtung a durch das betrachtete Raumelement R der Probe 1, ohne dass diese selbst bewegt werden muss.

Jeder Schnitt F1, F2 bzw. F3 liegt in einer Tiefe T1, T2 bzw. T3 der Probe 1, in welcher jeweils die Kohärenzbedingung erfüllt ist, so dass eine Interferenz zwischen dem vom Referenzspiegel 16 und dem von der Probe 1 reflektierten Licht auftreten kann. Die makroskopische Bewegung des Referenzspiegels 16 in Kombination mit der sukzessiven zweidimensionalen Erfassung des von der Probe 1 reflektierten Lichts hat somit die Wirkung eines dreidimensionalen Tiefenscans.

Die oben beschriebene Kombination der makroskopischen Linearbewegung des Referenzspiegels 16 einerseits mit der Erfassung des von der Probe 1 reflektierten Lichts mit einem zweidimensionalen Detektor 30 andererseits ermöglicht eine einfach zu realisierende und schnelle Aufnahme eines vollständigen dreidimensionalen Datensatzes des gewünschten Raumelements R der Probe 1.

Durch die makroskopische Bewegung des Referenzspiegels 16 wird hierbei ein dreidimensionales Tomogramm anstatt eines nur zweidimensionalen Bildes aus einer bestimmten Tiefe erhalten. Dabei braucht die Probe 1 zur Aufnahme eines dreidimensionalen Datensatzes relativ zum zweiten Interferometer 20 nicht mehr bewegt zu werden. Dies macht die beschriebene OCT-Einrichtung kompakt, zuverlässig und einfach handhabbar, so dass dieses besonders für den Einsatz in vivo geeignet ist.

Zur weiteren Veranschaulichung zeigt der linke Teil der Figur 5 ein Modell der menschlichen Haut, in welches beispielhaft ein Raumelement eingezeichnet ist, von welchem im Betriebsmodus 3 ein dreidimensionales Tomogramm aufgenommen wird.

### 3. Fokusnachführung

Die oben beschriebene OCT-Einrichtung ist so entworfen, dass während eines vollen Hubs, d.h. der Weglänge bzw. der doppelten Amplitude, der Bewegung des Referenzspiegels 16 stets ein Interferenzsignal mit ausreichend hoher Intensität und hoher Schärfe erhalten wird. Durch die nachfolgend näher beschriebene Fokusnachführung wird darüber hinaus gewährleistet, dass das Interferenzsignal sowie die Schärfe des erfassten Interferenzmusters für alle Tiefen in der Probe 1 maximal sind.

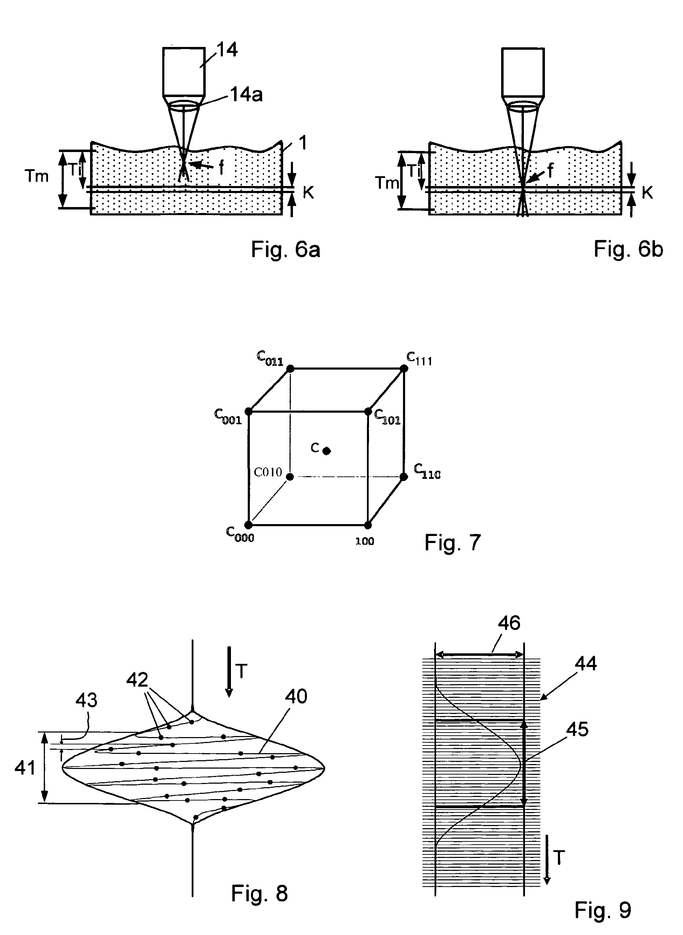

Dazu wird während der Erfassung des von der Probe 1 reflektierten Lichts der Fokus, d.h. der Brennpunkt, der im Probenarm 14 befindlichen Abbildungsoptik des Interferometers 10 in der Weise eingestellt, dass die Lage des Fokus in der Probe 1 und die Lage derjenigen Ebene in der Probe 1, bei welcher im Falle einer Reflexion von Licht die Kohärenzbedingung erfüllt ist und Interferenz auftritt, zu allen Zeiten während der Aufnahme eines Tomogramms des Raumelements R der Probe 1 im Wesentlichen identisch sind. Dies wird nachfolgend anhand der Figuren 6a und 6b veranschaulicht.

Figur 6a zeigt den Fall, bei welchem der Fokus f des - hier nur vereinfacht als Linse dargestellten - Probenobjektivs 14a im Probenarm 14 in einer Tiefe der Probe 1 liegt, die nicht mit der Lage des Kohärenz-Gates K übereinstimmt. Der innerhalb des Kohärenz-Gates K in der Tiefe Ti erfasste Schnitt durch die Probe 1 wird dadurch nicht exakt scharf auf den Detektor 30 (siehe Fig. 1) abgebildet, so dass Informationsverluste bei der Erfassung der Interferenz hinzunehmen wären.

In Figur 6b ist dagegen der Fall dargestellt, bei welchem der Fokus f des Probenobjektivs 14a derart eingestellt wurde, dass er innerhalb des Kohärenz-Gates K in der Tiefe Ti liegt. Dieses Nachführen des Fokus f des Probenobjektivs 14a entsprechend der jeweiligen Tiefe Ti des Kohärenz-Gates K wird als Fokus Tracking bezeichnet. Auf diese Weise wird das Interferometer 10 während des Tiefenscans auf die jeweilige Lage des Kohärenz-Gates K in unterschiedlichen Tiefen Ti der Probe 1 scharf gestellt, so dass aus jeder Tiefe der Probe 1 Bilder mit hoher Schärfe erhalten werden.

Die maximale optische Scantiefe Tm gibt an, bis zu welcher Tiefe unterhalb der Oberfläche der Probe 1 die Kohärenzbedingung für eine konstruktive Interferenz erfüllt und entsprechende Interferenzmuster erhalten werden.

Das in den Figuren 6a und 6b vereinfacht dargestellte Probenobjektiv 14a umfasst vorzugsweise mehrere Linsen, die einzeln und/oder gruppenweise in Richtung auf die Probe 1 bzw. von dieser weg bewegt werden können. Hierzu ist z.B. ein piezoelektrischer Aktuator, insbesondere ein Ultraschall-Piezo-Motor, vorgesehen, der mit dem Probenobjektiv 14a bzw. den Linsen gekoppelt ist und dieses bzw. diese entlang einer oder mehrer Führungen, insbesondere Führungsstäbe oder Führungsnuten, bewegt.

Die Bewegung des Probenobjektivs 14a bzw. der Linsen erfolgt vorzugsweise synchron mit der makroskopischen Bewegung des Referenzspiegels 16 im Interferometer 10 (siehe Fig. 1). Auf diese Weise folgt der Fokus f des Probenobjektivs 14a dem Kohärenz-Gate G, während Letzteres sukzessive unterschiedliche Tiefen T1, T2 bzw. T3 der Probe 1 durchfährt, aus denen mit Hilfe des Detektors 30 jeweils zweidimensionale Schnitte F1, F2 bzw. F3 (vgl. Fig. 5) aufgenommen werden.

Die Synchronisation der makroskopischen Bewegung des Referenzspiegels 16 und der Fokusnachführung einerseits in Kombination mit einem zweidimensionalen Detektor 30 andererseits gewährleistet eine besonders einfache und schnelle Aufnahme einer Vielzahl von scharfen zweidimensionalen Bildschnitten in unterschiedlichen Tiefen der Probe 1 und damit die Erfassung eines vollen dreidimensionalen Bilddatensatzes mit hoher Bildqualität.

Da das Interferometer 10 und die optische Abbildung im Probenarm 14 kontinuierlich aufeinander abgestimmt werden, sind die vom Detektor 30 erfassten Interferenzsignale für jede Tiefe in der Probe 1 maximal, so dass sich ein sehr hohes Signal-zu-Rausch-Verhältnis ergibt. Darüber hinaus wird dadurch sichergestellt, dass die laterale Auflösung für alle Tiefen in der Probe 1 optimal ist, da der Fokus f der Abbildung stets im Kohärenz-Gate K liegt. Es werden dadurch detailgetreue OCT-Bilder mit hohem Kontrast erhalten.

Vorteilhafterweise ist die Geschwindigkeit v2 der Bewegung der Linse bzw. Linsen des Probenobjektivs 14a in Richtung der Probe 1 kleiner als die Geschwindigkeit v1 der Bewegung des Referenzspiegels 16. Vorzugsweise wird hierbei ein Verhältnis v1/v2 der Geschwindigkeiten des Referenzspiegels 16 und der Linsen gewählt, das näherungsweise gleich 2·n - 1 ist oder bis zu etwa ± 20 %, vorzugsweise bis zu etwa ± 10 %, um diesen Wert liegt. Hierdurch werden die Lage des Fokus f und Kohärenz-Gates G mit besonders hoher Zuverlässigkeit aufeinander abgestimmt.

Durch die oben beschriebene Wahl des Verhältnisses v1/v2 der Geschwindigkeiten des Referenzspiegels 12 und der Linsen 42 wird gewährleistet, dass das Kohärenz-Gate K und der Fokus f während des Tiefenscans im gesamten betrachteten Tiefenbereich übereinander liegen. Im obigen Beispiel einer Probe mit einem Brechungsindex n = 1,4 liegt das Verhältnis v1/v2 der Geschwindigkeiten im Bereich von etwa (2·1,4 - 1) ± 20 %, d.h. zwischen etwa 1,44 und 2,16, und beträgt vorzugsweise etwa 2·1,4 - 1 = 1,8.

### 4. Trilineare Interpolation

Die mit der oben beschriebenen OCT-Einrichtung bzw. Methode erhaltenen OCT-Bilder können zur weiteren Verbesserung der Erkennung diagnostischer Information, beispielsweise im Bereich der Dermatologie zur noch besseren Erkennung von Hohlräumen oder Verdickungen in der Haut mit einer Größe von mehr als etwa 10 µm, einer Interpolation unterzogen werden.

Eine bei den mit der oben beschriebenen OCT-Einrichtung bzw. Methode erhaltenen OCT-Bildern, insbesondere Echtzeitbildern, besonders vorteilhafte Interpolationsmethode stellt hierbei die sog. trilineare Interpolation dar, bei welcher die Ausgangsbildwerte von mindestens zwei zweidimensionalen Ausgangsbildern, die in parallel zueinander verlaufenden Ebenen des Objekts aufgenommen wurden, im dreidimensionalen Raum interpoliert werden, so dass ein zweidimensionales Endbild erhalten wird. Dies wird nachfolgend im Einzelnen näher erläutert.

Bei der trilinearen Interpolation handelt es sich um ein Verfahren zur multivarianten Interpolation in einem dreidimensionalen regelmäßigen Gitter, d.h. einem Gitter mit gleicher Gitterkonstante in allen drei Raumrichtungen. Dies wird anhand eines in Figur 7 beispielhaft gezeigten Gitters veranschaulicht. Aus einer Interpolation der an den acht Ecken C000 bis C111 eines Würfels befindlichen Ausgangsbildwerten wird jeweils ein im Mittelpunkt C des Würfels befindlicher Interpolationswert abgeleitet.

Die jeweiligen Ausgangsbildwerte stammen von in verschiedenen Ebenen des Objekts aufgenommenen Ausgangsbildern. Bei den Ausgangsbildwerten handelt es sich um Lichtintensitätswerte an unterschiedlichen Orten in den entsprechenden zweidimensionalen Ausgangsbildern. Beispielsweise stammen die Ausgangsbildwerte, d.h. die Lichtintensitätswerte, mit den Koordinaten C000, C001, C011 und C010 von einem im Betriebsmodus 1 aufgenommenen ersten Echtzeitbild entlang eines ersten Tiefenschnittes S (siehe Fig. 3) und die Ausgangsbildwerte, d.h. die Lichtintensitätswerte, mit den Koordinaten C100, C101, C111 und C110 von einem im Betriebsmodus 1 aufgenommenen zweiten Echtzeitbild entlang eines davon im Abstand der Gitterkonstante beabstandeten zweiten Tiefenschnittes S (siehe Fig. 3). In einem alternativen Beispiel stammen die Ausgangsbildwerte mit den Koordinaten C000, C010, C110 und C100 von einem im Betriebsmodus 2 aufgenommenen ersten Echtzeitbild in Form eines ersten zweidimensionalen Tomogramms F (siehe Fig. 4) und die Ausgangsbildwerte mit den Koordinaten C001, C011, C111 und C101 von einem im Betriebsmodus 2 aufgenommenen zweiten Echtzeitbild in Form eines im Abstand der Gitterkonstante davon beabstandeten zweiten zweidimensionalen Tomogramms F (siehe Fig. 4).

Für eine trilineare Interpolation der mit der oben beschriebenen OCT-Einrichtung bzw. Methode erhaltenen OCT-Bilder, insbesondere der Echtzeitbilder, wird eine identische Auflösung in allen drei Raumdimensionen gewählt.

Bei aus dem Stand der Technik bekannten OCT-Systemen ist dies nicht ohne Auflösungsverlust erreichbar, da meist nur eine relativ hohe axiale (d.h. longitudinale, in Richtung des auf das Objekt treffenden Lichts) Auflösung realisiert werden kann, wohingegen die laterale (d.h. transversale, senkrecht zur Richtung des auf das Objekt treffenden Lichts) Auflösung meist deutlich niedriger ist. Eine Wahl gleicher Auflösung in allen drei Raumrichtungen wäre daher nur durch eine Erniedrigung der axialen Auflösung möglich, was jedoch wegen des großen Informationsverlusts in der Regel nicht wünschenswert ist, da dann kleine Objekte nicht mehr aufgelöst werden könnten. Außerdem ist es bei aus dem Stand der Technik bekannten OCT-Systemen nicht möglich, zwei zweidimensionale Bilder gleichzeitig oder zumindest fast gleichzeitig aufzunehmen. Dies gilt insbesondere für En-face-Bilder und scannende Systeme. Eine trilineare Interpolation in Echtzeit ist dadurch nahezu unmöglich, da dann auch Bewegungsartefakte relevant werden.

Bei den mit der oben beschriebenen OCT-Einrichtung bzw. Methode erhaltenen OCT-Bildern ist dagegen eine trilineare Interpolation sowohl im Falle der in den Betriebsmodi 1 und 2 erfassten zweidimensionalen Echtzeitbildern (Slice bzw. En-face) als auch zur Nachbearbeitung der im statischen Betriebsmodus 3 erhaltenen dreidimensionalen Tomogrammen möglich.

Bei der oben beschriebenen OCT-Einrichtung wird die axiale (d.h. longitudinale) Auflösung im Wesentlichen durch die spektrale Bandbreite der Lichtquelle 20 und den Brechungsindex des zu untersuchenden Objekts 1 bestimmt, während die laterale (d.h. transversale) Auflösung im Wesentlichen durch die optische Abbildung und die Größe der Detektorelemente 31 des Detektors 30 (siehe Figuren 1 und 2) bestimmt wird.

Die oben beschriebene OCT-Einrichtung ist so abgestimmt, dass laterale und axiale Auflösung nahezu gleich und sehr hoch sind. Vorzugsweise liegt die Auflösung in allen drei Dimensionen bei ca. 3 µm x 3 µm x 3µm.

Dies wird für die laterale Auflösung insbesondere durch die oben beschriebene Fokusnachführung und für die axiale Auflösung insbesondere durch die Verwendung einer Lichtquelle 20 erreicht, die eine Halogenlampe als Strahlungsquelle 21 in Kombination mit einem Gauß-Filter 22 umfasst.

Es ist außerdem bevorzugt, dass die Schärfentiefe der abbildenden Optik, insbesondere des Probenobjektivs 14, des Interferometers 10 (siehe Figur 1) größer ist als der "Gitterabstand" der Ausgangsbildwerte, d.h. der räumliche Abstand der Ausgangsbildwerte in den drei Dimensionen. Dadurch wird in jedem Fall gewährleistet, dass die Ausgangsbildwerte stets mit hoher Genauigkeit erfasst werden.

Vorzugsweise wird darüber hinaus der Tatsache Rechnung getragen, dass die Abtastung des Interferenzsignals hoch genug sein muss, um das sog. Abtasttheorem nicht zu verletzen. Dies wird nachfolgend näher erläutert.

Figur 8 zeigt ein Schema zur Veranschaulichung einer Abtastung eines Interferenzmusters 40 in Richtung der Tiefe T eines Objekts im Vergleich zur physikalischen Auflösung 41 in Richtung der Tiefe T. Bei der oben beschriebenen OCT-Einrichtung bzw. Methode werden vorzugsweise jeweils vier Punkte 42 pro Interferenzperiode des Interferenzmusters 40 abgetastet. Eine Interferenzperiode ist hierbei eine halbe (mittlere) Wellenlänge des in das Interferometer eingekoppelten Lichts lang (bei einer mittleren Wellenlänge von ca. 1,3 µm entspricht dies ca. 0,65 µm). Daraus folgt, dass der Abstand 43 zweier Abtastpunkte 42 ca. 0,163 µm beträgt. Die physikalische Auflösung 41 in Luft beträgt jedoch etwa 4 µm. Dies bedeutet, dass etwa 24 aufeinander folgende Zeilen in Tiefenrichtung T annähernd dieselbe physikalische Information enthalten und daher ohne signifikanten Informationsverlust zu einer Zeile zusammengefasst werden können. Dies wiederum hat zur Folge, dass der resultierende Volumenbildpunkt (sog. Voxel) in allen drei Dimensionen nahezu gleiche Ausdehnung hat, also im Wesentlichen einem Würfel entspricht. Der Ausgangsbildwert entspricht dabei beispielsweise einem Mittelwert oder dem Median aus den ursprünglichen Ausgangsbildwerten.

Figur 9 veranschaulicht das oben beschriebene Zusammenfassen von in Richtung der Tiefe T des Objekts in mehreren aufeinanderfolgenden Zeilen 44 abgetasteten ursprünglichen Ausgangsbildwerten zu einer Zeile mit nur einem Ausgangsbildwert und einer Zeilenhöhe, d.h. einer longitudinalen Ausdehnung 45 in Tiefenrichtung T, die der lateralen Ausdehnung 46 eines Bildpunktes (Pixels) der Zeile senkrecht zur Tiefenrichtung T entspricht.

Im Betriebsmodus 1, bei welchen Slices in Echtzeit aufgenommen werden, werden bei der trilinearen Interpolation zwei benachbarte Zeilen des Detektors 30 gleichzeitig ausgelesen. Im Beispiel des in Figur 2 gezeigten Detektors 30 bedeutet dies, dass die Breite b2 der Teilfläche A2 des Detektors 30 so gewählt wird, dass sich diese in Richtung der Breite des Detektors 30 nur über zwei Detektorelemente 31 erstreckt. Die Teilfläche A2 umfasst dann nur 2 x 640 Detektorelemente 31, die während einer makroskopischen Bewegung des Referenzspiegels16 (siehe Figur 1) sukzessive ausgelesen und in der oben beschriebenen Weise zu einem zweidimensionalen Endbild verrechnet werden.

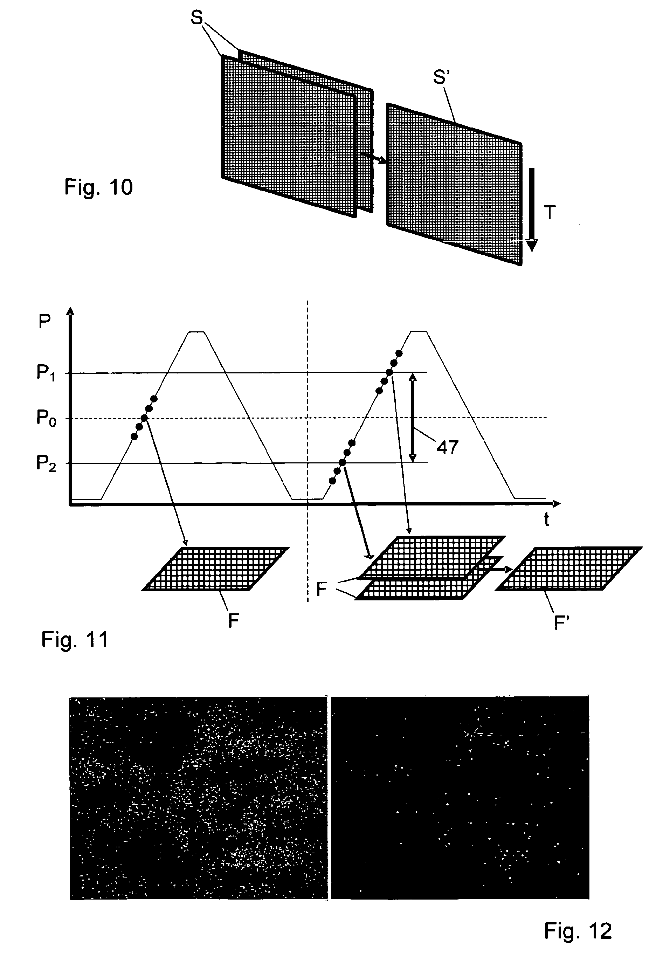

Dies ist anhand von Figur 10 veranschaulicht. Zwei in Richtung der Tiefe T des Objekts aufgenommene Ausgangsbilder S im Form von zwei Tiefenschnitten (vgl. Figur 3) werden durch trilineare Interpolation zu einem Endbild S' zusammengefasst.

Da die beiden Ausgangsbilder S in Form von zwei Tiefenschnitten gleichzeitig und in sehr kurzer Zeit aufgenommen werden, ist sichergestellt, dass eine etwaige Relativbewegung zwischen Sensorkopf und Objekt, insbesondere der menschlichen oder tierischen Haut, während der Aufnahme der beiden zweidimensionalen Ausgangsbilder S keine Rolle spielt.

Im Betriebsmodus 2, bei welchem En-face-Bilder in Echtzeit aufgenommen werden, führt der in einer mittleren Position befindliche Referenzspiegel 16 (siehe Figur 1) nur eine mikroskopische, vorzugsweise oszillierende, Bewegung von ca. +/- 5 µm bis +/- 40 µm durch. Die Position bzw. optische Abbildungseigenschaft des Probenobjektivs 14 ist hierbei vorzugsweise so eingestellt, dass dieses einen Brennpunkt in einer durch makroskopische Verschiebung des Referenzspiegels 16 vorgegebenen mittleren Tiefenposition aufweist. Bei der trilinearen Interpolation der in Echtzeit aufgenommenen En-face-Bilder werden - im Gegensatz zum Betrieb ohne trilineare Interpolation - jeweils zwei Ausgangsbilder in Form von zwei En-face-Bildern an zwei unterschiedlichen Positionen des Referenzspiegels 16 erfasst und zu einem zweidimensionalen Endbild in Form eines En-face-Bildes verrechnet.

Dies ist wird anhand des in Figur 11 gezeigten Diagramms veranschaulicht, welches den Verlauf der Position P des Referenzspiegels 16 über der Zeit t zeigt.

Im linken Teil des Diagramms der Figur 11 ist der Fall ohne trilineare interpolation dargestellt. Hierbei wird im Betriebsmodus 2 ein zweidimensionales Ausgangsbild F in Form eines Tomogramms aus einer bestimmten Tiefe im Objekt erhalten, indem bei fünf symmetrisch um eine mittlere Position P₀ gelegenen Positionen P des Referenzspiegels 16 gemessen wird.

Im rechten Teil des Diagramms der Figur 11 ist die Anwendung der trilinearen Interpolation veranschaulicht. Zwei zweidimensionale Ausgangsbilder F werden erhalten, indem bei jeweils fünf Positionen P des Referenzspiegels 16 gemessen wird. Die fünf Positionen P liegen jeweils symmetrisch um die Positionen P₁ bzw. P₂, welche vorzugsweise selbst symmetrisch um die mittlere Position P₀ des Referenzspiegels 16 liegen. Der Abstand 47 der Positionen P₁ und P₂ des Referenzspiegels 16 wird hierbei durch die axiale und/oder laterale Pixelgröße 45 bzw. 46 (siehe Figur 9) bestimmt. Bei der bevorzugt symmetrischen Lage der Positionen P₁ und P₂ liegen die entsprechenden Tomogramme F im Objekt jeweils um eine halbe Pixelgröße oberhalb bzw. unterhalb der mittleren Tiefenposition. Die auf diese Weise aufgenommenen beiden Ausgangsbilder F werden dann einer trilinearen Interpolation unterzogen, bei welcher das Endbild F' erhalten wird.

Vorzugsweise wird die Schärfentiefe der optischen Abbildung im Interferometer 10 (siehe Figur 1) so gewählt, dass diese größer ist als die halbe Voxelgröße. Bei einer bevorzugten Voxelgröße von 3 µm muss die Schärfentiefe also größer als 1,5 µm sein.

Da die beschriebene Erfassung der beiden Ausgangsbilder im Betriebsmodus 2 unmittelbar hintereinander erfolgt, typischerweise in einem zeitlichen Abstand von ca. 0,014 Sekunden, ist ein Einfluss einer etwaigen Relativbewegung zwischen Sensorkopf und Objekt, insbesondere der Haut, zwischen der Aufnahme der beiden ursprünglichen Ausgangsbilder auf das erhaltene Ausgangsbild nahezu ausgeschlossen oder vernachlässigbar klein.

Vorzugsweise steht der Sensorkopf bei der Aufnahme der Bilder in direktem Kontakt zur Oberfläche des zu untersuchenden Objekts, insbesondere der Haut, wodurch die Wahrscheinlichkeit einer Relativbewegung stark reduziert wird. Dies ist insbesondere bei Aufnahmen von Bildern der menschlichen oder tierischen Haut von besonderem Vorteil, da diese im Allgemeinen elastisch ist und, insbesondere bei Aufbringung eines Gels, an der Spitze des Sensorkopfes haftet, so dass leichte seitliche Bewegungen oder leichtes Verkippen des Sensorkopfes meist nicht zu einer Relativbewegung zwischen Haut und Sensorkopf führen.

Im Betriebsmodus 3, bei welchem statische dreidimensionale Tomogramme aufgenommen werden, wird - wie oben näher beschrieben - eine Schwebung zwischen der Detektorempfindlichkeitsmodulation einerseits und dem zu erfassenden Interferenzsignal andererseits erzeugt. Dadurch ist der Abstand der einzelnen Abtastpunkte in Tiefenrichtung größer als im Betriebsmodus 1, so dass entsprechend weniger Abtastpunkte, vorzugsweise zwischen 6 und 10, insbesondere 8, zusammengefasst werden, um ein würfelförmiges dreidimensionales Bildelement (Voxel) zu erhalten.

Figur 12 zeigt ein Beispiel eines Ausgangsbildes (links) im Vergleich zu einem entsprechenden Endbild (rechts), welches durch die beschriebene Interpolation erhalten wurde. Das Endbild ist gegenüber dem Ausgangsbild weniger stark verrauscht und erscheint daher "weicher" oder "glatter". Bei Vergleichen in der Interpretation der Bilder zu Diagnosezwecken hat sich, insbesondere im Bereich der Dermatologie, herausgestellt, dass den durch trilineare Interpolation erhaltenen Endbildern schneller und sicherer die jeweils relevante diagnostische Information entnommen werden kann. Dies gilt insbesondere für Hohlräume oder Inhomogenitäten mit einer Größe von typischerweise mehr als 10 µm.

Die obigen Ausführungen zur trilinearen Interpolation gelten auch für eine trikubische Interpolation entsprechend, bei welcher die Ausgangswerte nicht durch eine lineare Funktion, sondern durch eine kubische Funktion interpoliert werden.

### 5. System zur optischen Kohärenztomographie

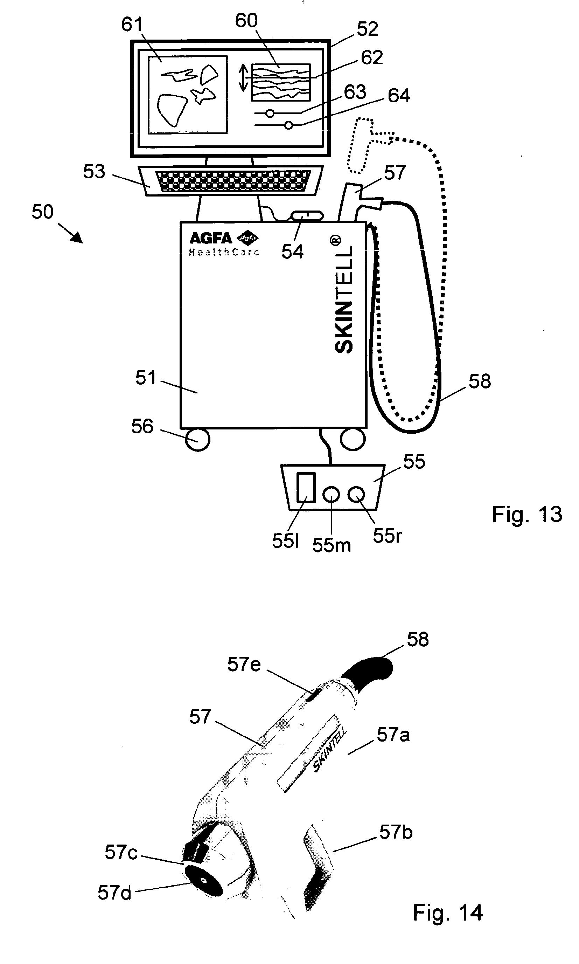

Figur 13 zeigt eine schematische Darstellung eines Systems 50 zur Durchführung des erfindungsgemäßen Verfahrens zur optischen Kohärenztomographie. Das System 50 umfasst ein Gehäuse 51, Eingabeeinrichtungen in Form einer Tastatur 53, einer Computermaus 54 sowie einer Fußschaltereinrichtung 55, welche einen linken, mittleren und rechten Fußschalter 55l, 55m bzw. 55r aufweist. Das Gehäuse 51 ist in dem dargestellten Beispiel fahrbar ausgestaltet, indem es mit Rollen 56 versehen ist.

Ferner ist ein Messkopf 57 vorgesehen, welcher über ein Kabel 58 oder einen Kabelschlauch oder -rohr mit dem Gehäuse 51 verbunden ist. In seiner Ruhestellung steckt der Messkopf 57 in einer am oder im Gehäuse 51 vorgesehenen Messkopfhalterung, aus welcher dieser während der Aufnahme von OCT-Bildern entnommen werden kann, was in der Figur durch den gestrichelt dargestellten Messkopf 57 bzw. das gestrichelt dargestellte Kabel 58 angedeutet ist.

Das System umfasst eine Anzeigeeinrichtung 52 in Form eines Flachbildschirms, auf welchem OCT-Bilder 60 und 61, welche durch Aufsetzen des Messkopfes 57 auf ein Objekt, insbesondere die Haut eines Patienten, erfasst wurden, dargestellt werden können. In dem in der Figur gezeigten Beispiel handelt es sich bei dem ersten OCT-Bild 60 um einen im Wesentlichen senkrecht zur Oberfläche des untersuchten Objekts verlaufenden Tiefenschnitt, der im oben beschriebenen Betriebsmodus 1 aufgenommen wurde, und bei dem zweiten OCT-Bild 61 um ein zweidimensionales Tomogramm, welches im Wesentlichen parallel zur Oberfläche des untersuchten Objekts verläuft und im oben beschriebenen Betriebsmodus 2 aufgenommen wurde.

Im Bereich des ersten OCT-Bildes 60 wird in der Anzeigeeinrichtung 52 eine Gerade 62 dargestellt, welche in Richtung des angedeuteten Doppelpfeils nach oben bzw. unten verschoben werden kann, beispielsweise indem mit Hilfe der Eingabeeinrichtungen 53, 54 bzw. 55 eine entsprechende Lage der Geraden 62 relativ zum ersten OCT-Bild 60 gewählt wird. Das System 50 ist so konfiguriert, dass entsprechend der gewählten Lage der Geraden 62 im dargestellten ersten OCT-Bild 60 eine senkrecht zum dargestellten ersten OCT-Bild 60 verlaufende Ebene im untersuchten Objekt automatisch ermittelt und dort ein zweidimensionales Tomogramm aufgenommen wird, welches dann als das zweite OCT-Bild 61 dargestellt wird.

Bei dem ersten OCT-Bild 60 handelt es sich vorzugsweise um einen sogenannten Slice, während das zweite OCT-Bild 61 vorzugsweise ein sogenanntes En-face-Bild darstellt, welches in einer der Geraden 62 im ersten OCT-Bild 60 entsprechenden Ebene aufgenommen worden ist.

Am Bildschirm der Anzeigeeinrichtung 52 wird ferner eine Tiefenauswahlanzeige 63 in Form eines entlang einer Geraden beweglichen Schaltersymbols dargestellt, welches die durch eine Wahl der Lage der Geraden 62 relativ zum dargestellten ersten OCT-Bild 60 ausgewählte Tiefe anzeigt. Alternativ oder zusätzlich kann die Tiefe auch in Form von Zahlenwerten angegeben werden.

In der Anzeigeeinrichtung 52 können ferner ein oder mehrere weitere Auswahlanzeigen vorgesehen sein. Im dargestellten Beispiel ist eine Auswahlanzeige 64 vorgesehen, welche eine oder mehrere Eigenschaften des zu untersuchenden Objekts anzeigt. Diese Eigenschaften werden vorzugsweise durch einen Bediener vor der Aufnahme entsprechender OCT-Bilder gewählt und eingegeben. Bei dermatologischen Anwendungen handelt es sich hierbei beispielsweise um einen Parameter zur Charakterisierung der Feuchtigkeit der Haut des jeweiligen Patienten. In der entsprechenden Auswahlanzeige 64 kann ein entsprechendes Schaltersymbol dann entlang einer Geraden kontinuierlich oder in vorgegebenen Schritten zwischen den Positionen "trockene Haut" links und "feuchte Haut" rechts bewegt werden.

In den Messkopf 57 ist das in Figur 1 dargestellte Interferometer 10 einschließlich der Optik 28 und des Detektors 30 integriert. Die Lichtquelle 20 einschließlich der eingangsseitigen Optik in Form der beiden Linsen 23 und 24 sind vorzugsweise im Gehäuse 51 des Systems 50 integriert. Der Lichtleiter 26, durch welchen die Lichtquelle 20 einerseits und das Interferometer 10 andererseits miteinander gekoppelt sind, wird in diesem Fall innerhalb des Kabels 58 vom Gehäuse 51 zum Messkopf 57 geführt. Im Kabel 58 werden darüber hinaus elektrische Leitungen geführt, welche einerseits zur Energieversorgung und Steuerung des Messkopfes 57 dienen und andererseits die bei der Erfassung von OCT-Bildern erzeugten Detektorsignale des Detektors 30 von diesem in das Gehäuse 51 leiten, wo sie einer Verarbeitungseinrichtung (nicht dargestellt) zugeführt werden.

Der in Figur 13 nur stark schematisiert gezeigte Messkopf 57 ist in Figur 14 im Detail dargestellt. Im unteren Bereich eines Messkopfgehäuses 57a des Messkopfes 57 ist ein Griff 57b vorgesehen, durch welchen der Messkopf 57 von einer Bedienperson aus der Messkopfhalterung am oder im Gehäuse 51 entnommen bzw. wieder in die Messkopfhalterung eingesteckt und bei der Aufnahme von OCT-Bildern auf das Objekt aufgesetzt und ggf. an diesem entlang geführt werden kann. Hierbei wird der Messkopf 57 mit einer am vorderen Ende des Messkopfgehäuses 57a befindlichen Kontaktfläche 57c in Kontakt mit dem zu untersuchenden Objekt, insbesondere der Haut eines Patienten, gebracht.

In der Mitte der Kontaktfläche 57c ist ein Fenster 57d vorgesehen, durch welches Licht aus dem Probenarm 14 des im Messkopf 57 befindlichen Interferometers 10 (siehe Figur 1) austreten und dabei das zu untersuchende Objekt bestrahlen kann. Das in unterschiedlichen Tiefen des Objekts reflektierte und/oder zurückgestreute Licht tritt durch das Fenster 57d wieder in den Probenarm 14 des Interferometers 10 ein und kann dort, wie oben bereits ausführlich dargestellt wurde, in Form von lnterferenzerscheinungen erfasst und ausgewertet werden.

Am Messkopfgehäuse 57a ist ferner eine Statusanzeigeeinrichtung 57e, vorzugsweise in Form einer Leuchtanzeige, vorgesehen, durch welche z.B. die Bereitschaft des Systems 50 und/oder des Messkopfes 57 zum Erfassen von OCT-Bildern angezeigt wird.

Im Bereich des hinteren Endes des Messkopfgehäuses 57a ist das Kabel 58, das auch als Kabelkanal oder -schlauch ausgebildet sein kann, an den Messkopf 57 angeschlossen.

Mit dem oben beschriebenen System 50 zur optischen Kohärenztomographie können drei und zweidimensionale Querschnittsbilder eines Objekts, insbesondere der menschlichen Haut, aufgenommen werden, wobei die Eindringtiefen in die menschliche Haut von bis zu etwa 1 mm erreicht werden können und die Größe der Fläche des untersuchten Hautbereichs typische Abmessungen von etwa 1,8 x 1,5 mm aufweist. Aufgrund der im beschriebenen System 50 verwendeten Infrarotstrahlung mit einer bevorzugten mittleren Wellenlänge von etwa 1,3 µm kann eine Strahlungsbelastung des Patienten, wie beispielsweise bei der Verwendung von Röntgenstrahlung, ausgeschlossen werden. Die mit dem beschriebenen System 50 erfassten OCT-Bilder haben ferner eine hohe Auflösung und erlauben eine Darstellung von einzelnen Objektstrukturen mit einer Größe von bis zu 3 µm. Nicht zuletzt können die mit dem System 50 erfassten OCT-Bilder dazu verwendet werden, die absolute geometrische Ausdehnung der unterschiedlichen Strukturen, d.h. deren Größe, zu messen.

Das System 50 umfasst - wenn auch nicht explizit gezeigt - eine Steuerungseinrichtung zur erfindungsgemäßen Steuerung des Systems 50, insbesondere der optischen Kohärenztomographieeinrichtung, bzw. zur Durchführung der vorstehend und nachfolgend beschriebenen Abläufe. Das System umfasst ferner eine Verarbeitungseinrichtung zur Verarbeitung verschiedener Daten einschließlich der oben beschriebenen Interpolation von Ausgangsbildwerten. Die Steuerungseinrichtung und/oder die Verarbeitungseinrichtung sind vorzugsweise im Gehäuse 51 des Systems 50 integriert.

### 6. Workflows, Tiefen- und laterale Navigation

Im Folgenden werden die Funktionsweise und die Handhabung des Systems 50 zur optischen Kohärenztomographie anhand typischer und/oder bevorzugter Abläufe (sogenannter Workflows) beispielhaft beschrieben. Dabei werden die hierbei erzielten Vorteile dargelegt.

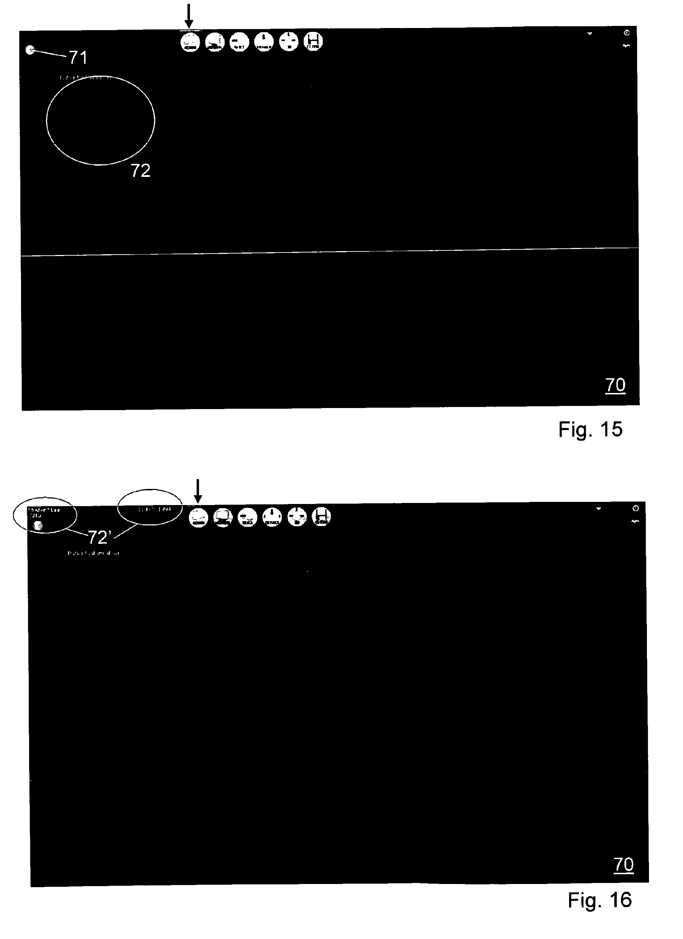

Figur 15 zeigt den Inhalt des Bildschirms 70 der Anzeigeeinrichtung im Verwaltungsmodus, in welchem sich das System nach dem Start automatisch befindet. Eine Statusanzeige 71 in Form eines geeigneten Symbols, beispielsweise einer grünen Kreisscheibe, zeigt die Bereitschaft des Systems an. Vorzugsweise wird die Bereitschaft des Systems, insbesondere zur Aufnahme von OCT-Bildern, gleichzeitig durch Aktivierung der am Messkopf 57 vorgesehenen Statusanzeige 57e angezeigt. Eine Bedienperson hat somit die Möglichkeit, die Bereitschaft des Systems alleine anhand der Statusanzeige 71 auf dem Bildschirm 70 oder anhand der Statusanzeige 57e am Messkopf 57 zu erkennen.

In einem Eingabefeld 72 können Informationen zu dem zu untersuchenden Objekt, insbesondere zu einem Patienten, eingegeben werden. Vorzugsweise ist hierbei das System so konfiguriert, dass eine Aufnahme von OCT-Bildern erst dann möglich ist, wenn zumindest eine der im Eingabefeld 72 geforderten Informationen eingegeben wird, beispielsweise zumindest der Nachname eines Patienten.

Die im Eingabefeld 72 eingegebenen Informationen, insbesondere Vor- und Nachname, Patientenidentifikationsnummer sowie Geburtsdatum, erscheinen sodann - wie in Figur 16 beispielhaft veranschaulicht - in entsprechenden Feldern 72' im oberen Bereich der Bildschirmanzeige 70.

Sobald der Messkopf 57 aus der am bzw. im Gehäuse 51 des Systems befindlichen Messkopfhalterung entnommen wird, startet das System automatisch im Betriebsmodus 1 (sogenannter Slice-Modus). Bevor die Kontaktfläche 57c des Messkopfes 57 in Kontakt mit der Haut des Patienten gebracht wird, wird auf die Kontaktfläche 57c des Messkopfes 57 ein optisches Gel aufgebracht, welches einerseits dafür sorgt, dass scharfe Brechungsindexübergänge zwischen der Haut und dem Fenster 57d des Messkopfes 57 überbrückt werden (sogenanntes Index-Matching) und andererseits Unebenheiten an der Hautoberfläche ausgeglichen werden. Vorzugsweise beträgt die Menge des auf die Kontaktfläche 57c aufgebrachten optischen Gels je nach Anwendungsfall zwischen etwa 2 µl und 10 µl.

Nach Aufbringen des Gels wird die Kontaktfläche 57c des Messkopfes 57 von einer Bedienperson gegen den zu untersuchenden Hautbereich des Patienten gedrückt und geringfügig auf dem Hautbereich hin und her bewegt, um eine günstige Verteilung des optischen Gels zu erreichen.

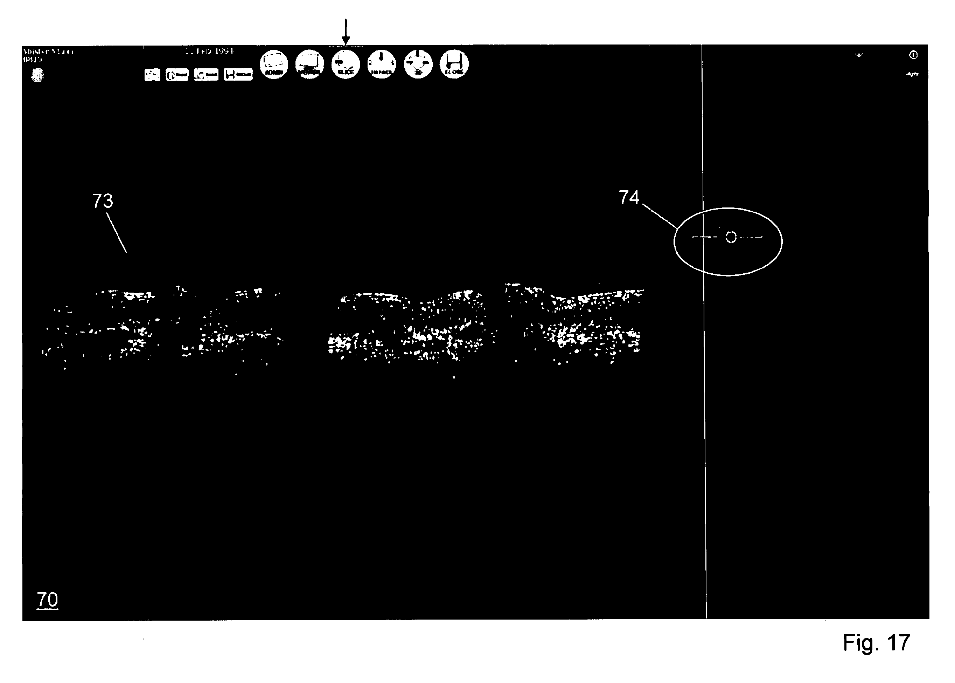

Da sich das System bereits unmittelbar nach dem Herausnehmen des Messkopfes 57 aus dem Messkopfhalter im Slice-Modus befindet, wird unmittelbar nach Herstellung eines Kontaktes mit dem zu untersuchenden Hautbereich ein Slice-Bild 73 erfasst und im Bereich der Mitte des Bildschirms 70 dargestellt, wie in Figur 17 veranschaulicht ist. Im rechten Bereich des Bildschirms 70 ist eine Anzeige 74 vorgesehen, in welcher der Hauttyp der jeweils untersuchten Haut eingestellt bzw. angezeigt werden kann. Vorzugsweise handelt es sich hierbei um einen den Feuchtigkeitsgehalt des untersuchten Hautbereichs charakterisierenden Parameter. Ein entsprechendes Schaltersymbol kann in diesem Fall von der Bedienperson auf einer Skala zwischen "trockene Haut" und "feuchte Haut" in Stufen oder auch stufenlos bewegt werden. Durch die Wahl dieses Parameters wird das Verhältnis der Geschwindigkeiten, mit welchen sich der Referenzspiegel 16 bzw. die Linse oder Linsen des Probenobjektivs 14a (siehe Figur 1 sowie Figuren 6a und 6b) bewegen, festgelegt, um eine optimale Fokusnachführung zu gewährleisten.

Bei der Aufnahme des in Figur 17 dargestellten Slice-Bildes 73 wurde eine etwas oberhalb der Mitte der Skala liegende Position des Schaltersymbols der Anzeige 74 gewählt, welche einer eher feuchteren Haut entspricht. Als Ergebnis wird ein helles und relativ kontrastreiches Slice-Bild 73 erhalten.

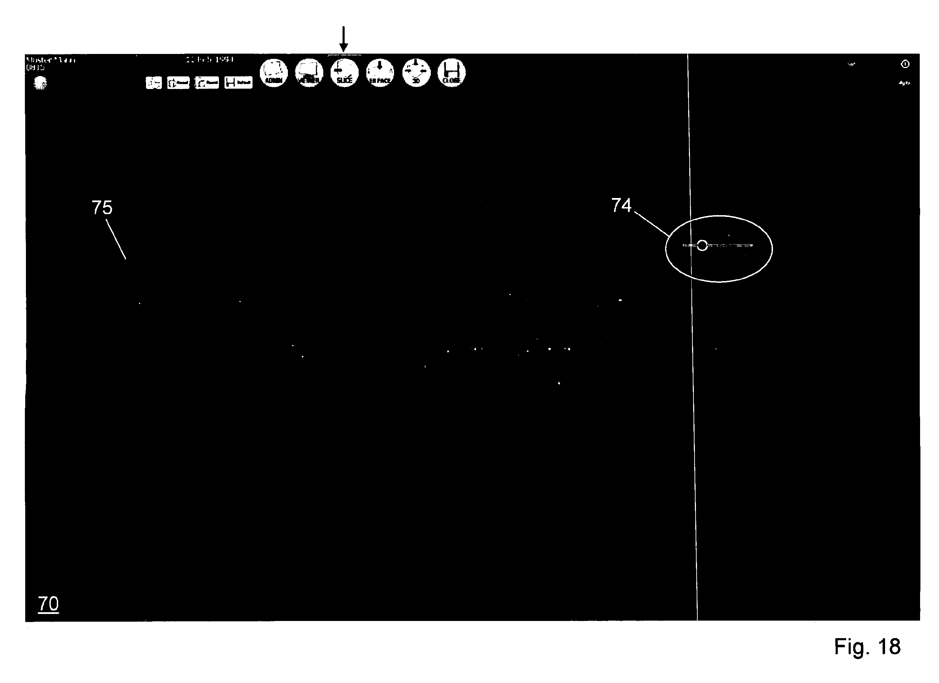

Im Gegensatz dazu wurde das in Figur 18 dargestellte Slice-Bild 75 bei einer Parametereinstellung erfasst, bei welcher das Schaltersymbol der Anzeige 74 unterhalb der Mitte der Skala liegt, was einer eher trockneren Haut entspricht. Wie in Figur 18 deutlich zu erkennen ist, ist der Kontrast des bei dieser Einstellung aufgenommenen Slice-Bildes 75 gegenüber dem in Figur 17 gezeigten Slice-Bild 73 wesentlich geringer. Dies kann damit erklärt werden, dass bei der Aufnahme des Slice-Bildes 75 in unterschiedlichen Tiefen der Haut der Fokus des Probenobjektivs 14 nicht oder nicht immer im Bereich des jeweiligen Kohärenz-Gates gelegen hat. Zu weiteren Einzelheiten wird auf die obigen Ausführungen im Zusammenhang mit der Fokusnachführung verwiesen.

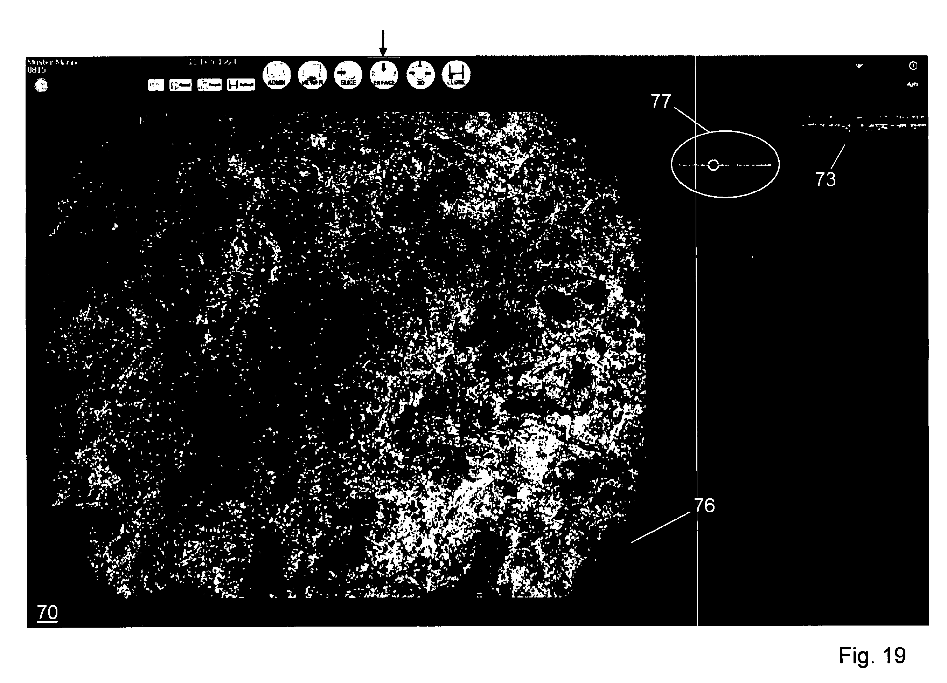

Ausgehend von einem bei optimaler Einstellung des der Hautfeuchtigkeit entsprechenden Parameters erhaltenen Slice-Bildes 73 (siehe Figur 17) kann durch Betätigen eines entsprechenden Schalters, vorzugsweise durch ein langes Drücken des mittleren Fußschalters 55m (siehe Figur 13), vom Slice-Modus in den En-face-Modus gewechselt werden, in welchem - wie in Figur 19 veranschaulicht wird - das Slice-Bild 73 im rechten Bereich des Bildschirms 70 verkleinert dargestellt (sogenannter Thumbnail) und gleichzeitig ein im Betriebsmodus 2, dem sogenannten En-face-Modus, aufgenommenes En-face-Bild 76 im mittleren Bereich des Bildschirms 70 gezeigt wird. Bei dem dargestellten En-face-Bild 76 handelt es sich vorzugsweise um ein Echtzeit-Bild, das mit einer Wiederholungsrate von mindestens einem Bild pro Sekunde aufgenommen und aktualisiert wird. Bei der verkleinerten Darstellung des Slice-Bildes 73 im rechten Bereich des Bildschirms 70 handelt es sich dagegen um ein statisches Bild, welches beispielsweise dem letzten im Slice-Modus (siehe Figur 17) in Echtzeit aufgenommen und dargestellten Slice-Bild entspricht.

Die Tiefe in der Haut, in welcher das dargestellte En-face-Bild 76 aufgenommen wird, kann durch die Bedienperson über einen am Bildschirm 70 angezeigten Tiefenauswahlschalter 77 ausgewählt werden, indem ein entsprechendes Schaltersymbol beispielsweise mit Hilfe der Computermaus 54, der Tastatur 53 und/oder der Fußschaltereinrichtung 55 (siehe Figur 13) betätigt wird. Vorzugsweise erfolgt die Einstellung bzw. Auswahl einer bestimmten Tiefe durch den linken Fußschalter 55l, welcher als Wippenschalter ausgebildet ist und durch ein Betätigen der Wippe nach vorne bzw. hinten eine Tiefenänderung hin zu einer größeren bzw. kleineren Tiefe veranlasst.

Die oben beschriebene Auswahl einer bestimmten Tiefe, in welcher ein zweites OCT-Bild aufgenommen wird, anhand eines dargestellten ersten OCT-Bildes, wird im Zusammenhang mit der Darstellung des erfindungsgemäßen Systems bzw. Verfahrens auch als Tiefennavigation bezeichnet.

Vorzugsweise ist das System so konfiguriert, dass eine Auswahl der Tiefe, in welcher ein En-face-Bild erfasst werden soll, bis zu einem Mikrometer genau erfolgen kann. Grundsätzlich ist es möglich, dass die Größe der Schritte, in welcher eine Tiefennavigation vorgenommen werden kann, vorgegeben wird. So kann beispielsweise vor Beginn einer Untersuchung, d.h. einer Aufnahme von mehreren OCT-Bildern bei einem Patienten, festgelegt werden, dass die Wahl der jeweiligen Tiefe für En-face-Bilder in Schritten von 5 µm erfolgen soll. Auf diese Weise kann die Tiefennavigation individuell an den jeweiligen Diagnosezweck angepasst werden.

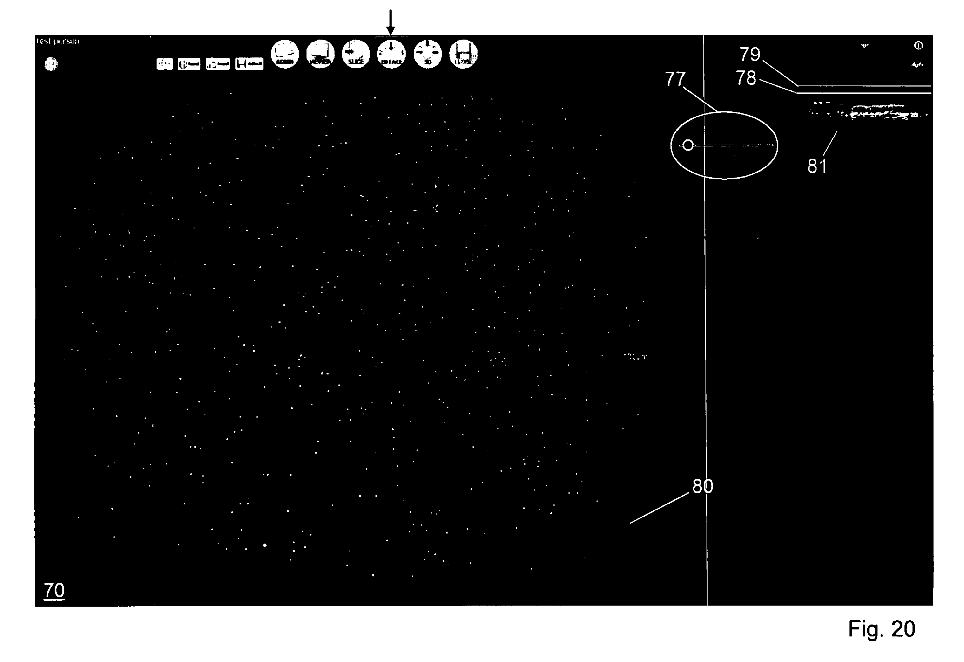

Die vorstehend beschriebene Wahl einer bestimmten Tiefe für die Aufnahme eines En-face-Bildes wird nachfolgend anhand von in den Figuren 20 bis 23 dargestellten Bildschirmanzeigen näher erläutert.

Figur 20 zeigt ein En-face-Bild 80, welches in einer Tiefe aufgenommen worden ist, welche zwischen dem am Messkopf 57 befindlichen Fenster 57d, welches in Form einer waagerechten Linie 79 im entsprechenden Slice-Bild 81 erkennbar ist, und der Hautoberfläche liegt und aus diesem Grund lediglich einen Querschnitt durch die zwischen dem Fenster 57d und der Haut befindliche Gelschicht zeigt. Die in diesem Beispiel eingestellte Tiefe wird mittels einer im verkleinert dargestellten Slice-Bild 81 eingezeichneten waagerechten Gerade 78 angezeigt. Darüber hinaus ist die gewählte bzw. eingestellte Tiefe auch der jeweiligen Stellung des Tiefenauswahlschalters 77 und/oder einer entsprechenden Zahlenwertanzeige zu entnehmen.

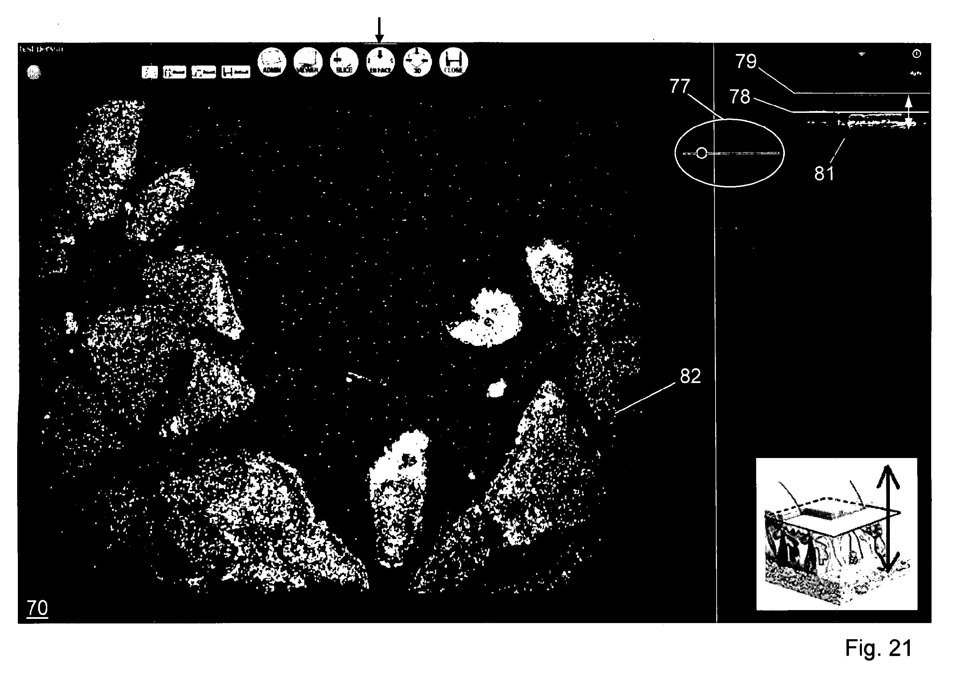

Figur 21 zeigt ein En-face-Bild 82, das in einer Ebene aufgenommen worden ist, welche im obersten Bereich der Hautoberfläche liegt, wie an der Lage der als Tiefenanzeige dienenden Geraden 78 relativ zum verkleinert dargestellten Slice-Bild 81 zu erkennen ist. Der Bereich zwischen der Geraden 78 einerseits und der horizontalen Linie 79, die von Lichtrefflektionen am Fenster 57d des Messkopfes 57 herrührt, andererseits entspricht der zwischen dem Fenster 57d und der Haut befindlichen Gelschicht.

Durch Betätigung des linken Fußschalters 55l bzw. des entsprechenden Schaltsymbols in der Tiefenauswahlanzeige 77, beispielsweise mit Hilfe der Computermaus54, kann die Gerade 78 relativ zum verkleinert dargestellten Slice-Bild 81 bewegt (siehe Doppelpfeil) werden, wodurch in unterschiedlichen Tiefen in der Haut liegende Ebenen ausgewählt werden können, in denen entsprechende En-face-Bilder aufgenommen und in der Bildschirmdarstellung angezeigt werden.

Das Prinzip der Tiefennavigation ist im rechten unteren Teil der Figur 21 anhand einer in ein Hautmodell eingezeichneten Ebene, die im Wesentlichen parallel zur Hautoberfläche verläuft und in Doppelpfeilrichtung in unterschiedliche Tiefen verschoben werden kann, weiter veranschaulicht.

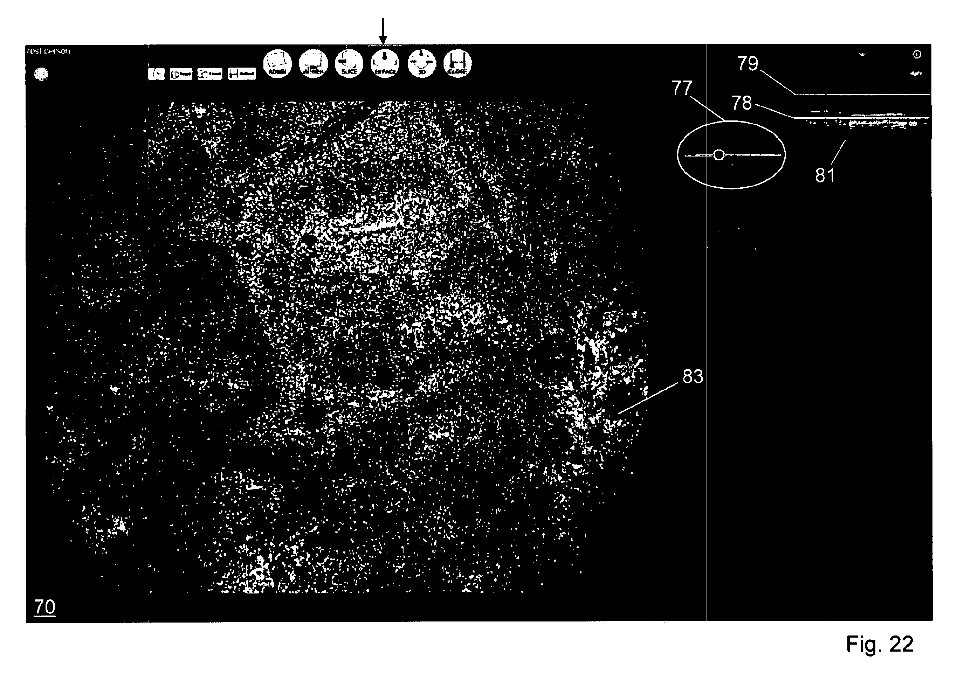

Figur 22 zeigt beispielhaft ein weiteres En-face-Bild 83, das in einer weiteren Tiefe des untersuchten Hautbereichs aufgenommen wurde. Wie anhand der Lage der Geraden 78 relativ zum Slice-Bild 81 zu erkennen ist, liegt die Ebene des erhaltenen En-face-Bildes 83 nunmehr vollständig innerhalb des untersuchten Hautbereichs. Im Übrigen gelten die Ausführungen im Zusammenhang mit den Figuren 20 und 21 entsprechend.

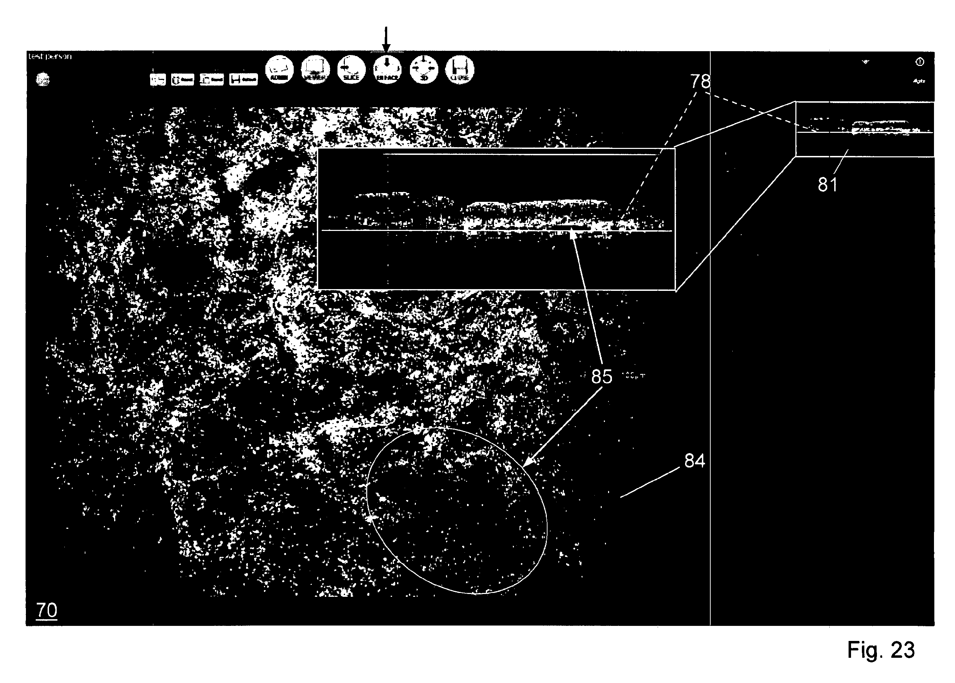

Figur 23 zeigt eine vorteilhafte Verwendung der oben beschriebenen Tiefennavigation beim Auffinden diagnostisch relevanter Informationen. So kann in dem dargestellten Slice-Bild 81 durch die Wahl der Lage der Geraden 78 die Tiefe bei der Aufnahme eines En-face-Bildes 84 gewählt werden, um beispielsweise einen anhand des Slice-Bildes 81 vermuteten Hohlraum 85 in einer entsprechenden, senkrecht zum Slice-Bild 81 verlaufenden Ebene des En-face-Bildes 84 näher zu analysieren.

Im Folgenden werden weitere Aspekte des beschriebenen Ablaufs bei der Aufnahme von OCT-Bildern mit dem erfindungsgemäßen System näher erläutert.

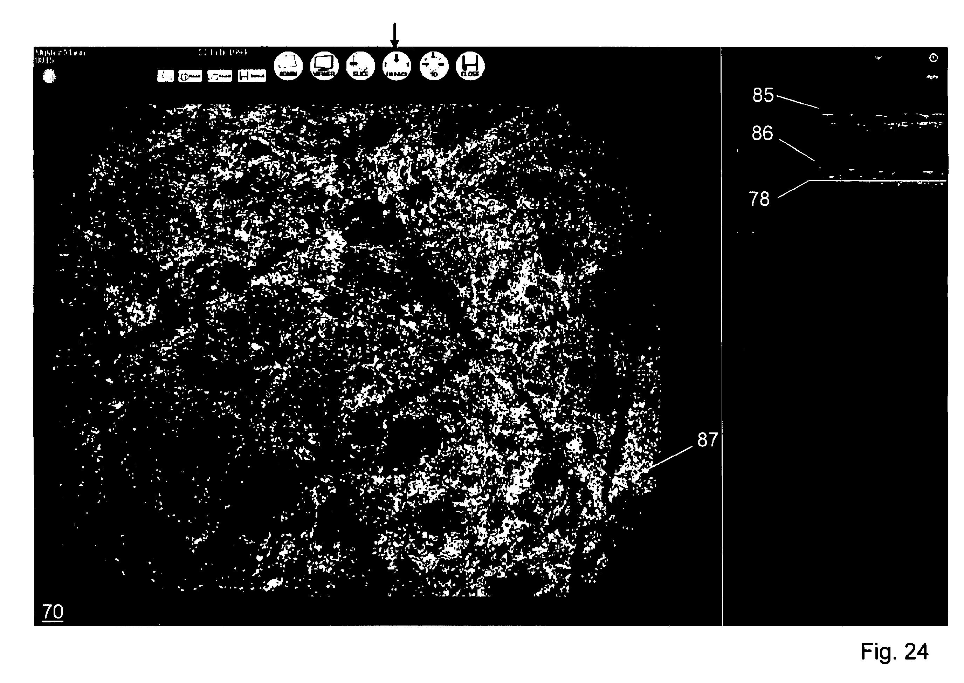

Im rechten Bereich des in Figur 24 gezeigten Bildschirms 70 ist ein Slice-Bild 85 verkleinert dargestellt, welches im Slice-Mode aufgenommen und durch Betätigen eines entsprechenden Schalters, vorzugsweise durch kurzes Drücken des mittleren Fußschalters 55m (siehe Figur 13), in einem nicht-flüchtigen Speicher des Systems, z.B. einem Festplattenspeicher, gespeichert wurde.

Anhand des gespeicherten und dargestellten Slice-Bildes 85 kann eine Tiefennavigation, wie sie im Zusammenhang mit den Figuren 19 bis 23 ausführlich dargestellt worden ist, durchgeführt werden. Vorzugsweise ist hierbei das System in der Weise konfiguriert, dass automatisch ein weiteres Slice-Bild 86 erzeugt und im rechten Bereich des Bildschirms 70 dargestellt wird, wenn das zuletzt gespeicherte Slice-Bild, in diesem Fall das Slice-Bild 85, bei einem Wechsel vom Slice-Modus in den En-face-Modus bereits älter als eine vorgegebene Zeitdauer, beispielsweise 10 Sekunden, ist. Dieser Fall ist in dem in Figur 24 dargestellten Beispiel gezeigt, bei welchem zu einem ersten Zeitpunkt das Slice-Bild 85 aufgenommen und nach Eingabe eines entsprechenden Benutzerbefehls gespeichert wurde und erst nach einer Zeitspanne von mehr als 10 Sekunden nach der Aufnahme bzw. Speicherung des Slice-Bildes 85 in den En-face-Modus gewechselt wurde. In diesem Fall wurde unmittelbar nach dem Wechsel in den En-face-Modus ein weiteres Slice-Bild 86 aufgenommen, temporär, z.B. in einem flüchtigen Speicher des Systems, gespeichert und im rechten Bereich des Bildschirms 70 verkleinert dargestellt, wobei zur Durchführung der oben beschriebenen Tiefennavigation im Bereich des OCT-Bildes 86 eine Gerade 78 eingeblendet wird, anhand welcher eine Bedienperson erkennen bzw. steuern kann, aus welcher Tiefe im Objekt jeweils ein entsprechendes En-face-Bild 87 aufgenommen und, vorzugsweise im mittleren Bereich des Bildschirms 70, dargestellt wird.

Durch diese Konfiguration des Systems wird gewährleistet, dass die oben beschriebene Tiefennavigation stets an einem möglichst aktuellen Slice-Bild vorgenommen wird, so dass etwaige Relativbewegungen zwischen dem Messkopf einerseits und dem Objekt andererseits einschließlich Bewegungen im Objekt selbst berücksichtigt werden können und damit die Zuverlässigkeit der Aufnahme von OCT-Bildern, insbesondere En-face-Bildern, nicht negativ beeinflussen können.

Der einstellbare Zeitraum zwischen der Aufnahme und Speicherung eines Slice-Bildes einerseits und dem Wechsel vom Slice-Modus in den En-face-Modus andererseits, bei dessen Überschreiten ein weiteres Slice-Bild aufgenommen, temporär gespeichert und am Bildschirm 70 angezeigt wird, wurde im dargestellten Beispiel auf 10 Sekunden festgelegt. Grundsätzlich ist es aber auch möglich, diesen Zeitraum deutlich kürzer, z.B. 5 Sekunden, zu wählen, falls dies die Art der jeweiligen Untersuchung erfordert. Dies kann beispielsweise der Fall sein, wenn der Messkopf aufgrund größerer Bewegungen des Objekts, insbesondere eines Patienten, nicht hinreichend lange in einer festen Position relativ zum Objekt gehalten werden kann. Andererseits ist es aber auch möglich, längere Zeiträume, z.B. 15 Sekunden, vorzugeben, wenn beispielsweise das zu untersuchende Objekt über einen längeren Zeitraum unbeweglich bleibt und eine feste Relativposition zwischen Messkopf und Objekt gewährleistet werden kann.

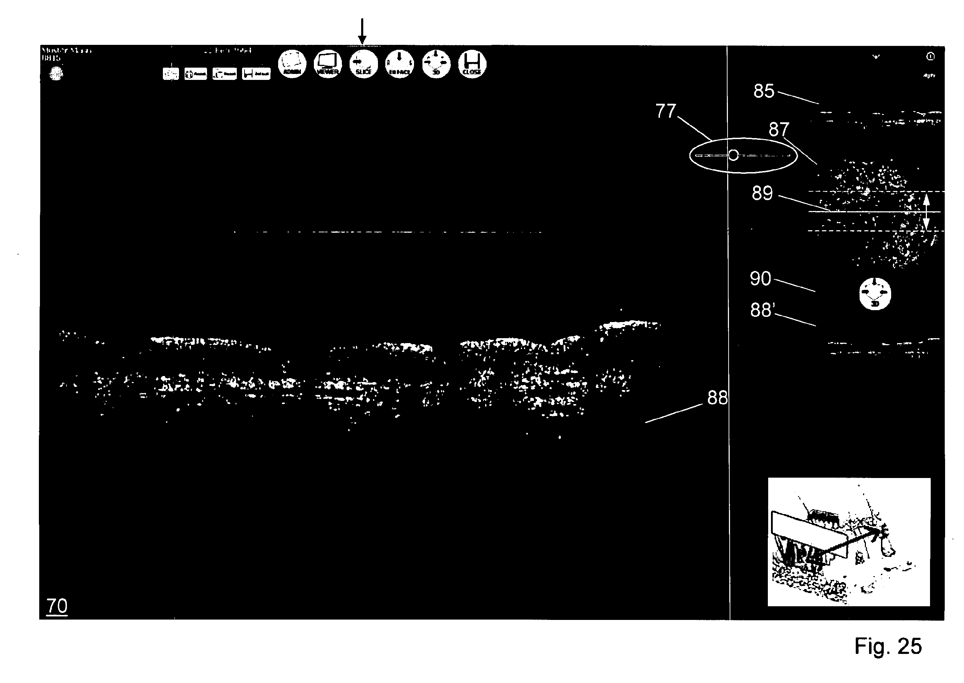

Figur 25 zeigt eine Bildschirmdarstellung 70, nachdem aus dem En-face-Modus, dessen Bildschirmanzeige beispielhaft in Figur 24 dargestellt ist, wieder in den Slice-Modus gewechselt wurde. Im rechten Bereich der Bildschirmdarstellung 70 wird in diesem Fall das aufgrund eines Benutzerbefehls permanent gespeicherte Slice-Bild 85 angezeigt, nicht jedoch das zu Navigationszwecken nur temporär gespeicherte Slice-Bild 86 (siehe Figur 24). Darüber hinaus wird nach dem Wechsel in den Slice-Modus das zuletzt im En-face-Modus aufgenommene und angezeigte En-face-Bild 87 in verkleinerter Form dargestellt.

Im mittleren Bereich des Bildschirms 70 wird ein aktuell aufgenommenes Slice-Bild 88 dargestellt. Analog zur oben beschriebenen Tiefennavigation ist das System so konfiguriert, dass auch in dem verkleinert dargestellten En-face-Bild 87 mit Hilfe einer zusätzlich dargestellten Geraden 89 eine Ebene senkrecht zur Ebene des dargestellten En-face-Bildes 87 ausgewählt werden kann, in welcher das Slice-Bild 88 aufgenommen wird.

Die anhand eines En-face-Bildes vorgenommene Auswahl von Slice-Bild-Ebenen, die im Wesentlichen parallel zu dem auf das Objekt treffenden Licht bzw. senkrecht zur Hautoberfläche oder zur Ebene des En-face-Bildes verlaufen, kann auch als laterale Navigation bezeichnet werden. Im Übrigen gelten die obigen Ausführungen im Zusammenhang mit der Tiefennavigation entsprechend.

Das Prinzip der lateralen Navigation ist im rechten unteren Teil der Figur 25 anhand einer in ein Hautmodell eingezeichneten Ebene, die im Wesentlichen senkrecht zur Hautoberfläche verläuft und in Doppelpfeilrichtung lateral verschoben werden kann, weiter veranschaulicht.

In der in Figur 25 gezeigten Bildschirmdarstellung 70 wird darüber hinaus im rechten Bereich ein Slice-Bild 88' dargestellt, welches im aktuell gewählten Slice-Modus durch einen entsprechenden Renutzerauswahlbefehl gespeichert wurde. Darüber hinaus wird ein 3D-Symbol 90 angezeigt, welches darauf hinweist, dass zwischenzeitlich auch ein im Betriebsmodus 3 aufgenommenes dreidimensionales Tomogramm aufgenommen und abgespeichert worden ist.

### 7. Bildbetrachtungs- und Verwaltungsmodus

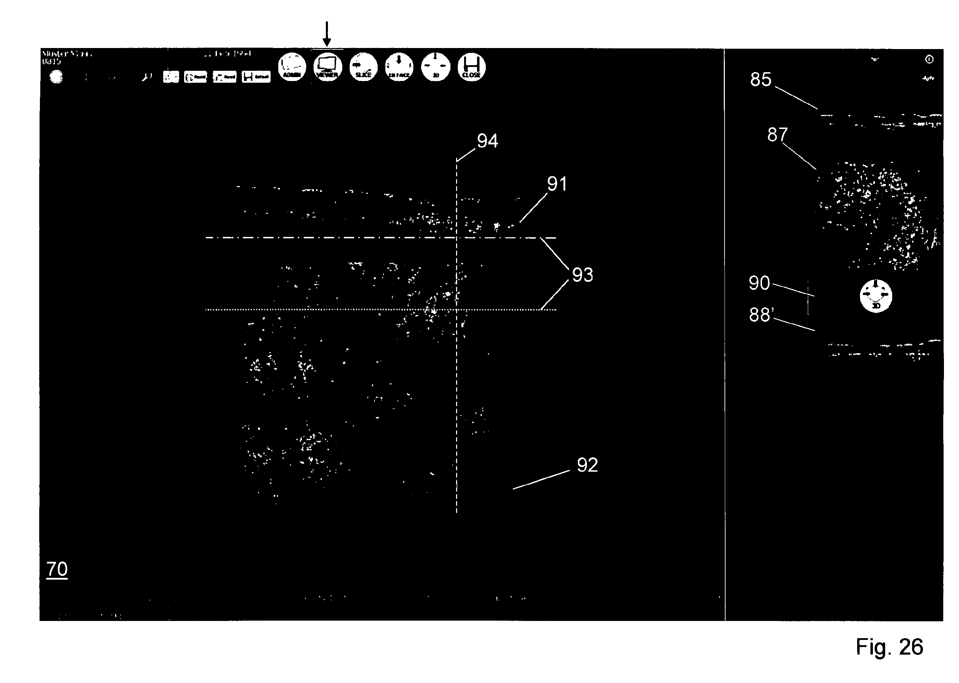

Nach Beendigung der Aufnahme eines oder mehrerer, gegebenenfalls unterschiedlicher, OCT-Bilder wird der Messkopf 57 wieder in die am Gehäuse 51 des Systems 50 befindlichen Messkopfhalterung eingesteckt, woraufhin die Bildschirmanzeige 70 - wie in Figur 26 dargestellt - automatisch in einen Bildbetrachtungsmodus übergeht, in welchem der Bediener die im rechten Bereich des Bildschirms 70 verkleinert dargestellten, gespeicherten OCT-Bilder 85, 87, 88' bzw. 90 auswählen kann, wobei das jeweils ausgewählte verkleinerte Bild im Bereich der Mitte des Bildschirms 70 vergrößert dargestellt wird.

Im Falle einer Auswahl des dreidimensionalen Tomogramms 90 kann im Bereich der Mitte des Bildschirms 70 eine perspektivische Wiedergabe des aufgenommenen dreidimensionalen Tomogramms erfolgen. Bei bestimmten diagnostischen Anwendungen kann es jedoch vorteilhafter sein, jeweils ein dem dreidimensionalen Tomogramm entstammendes Slice-Bild 91 und En-face-Bild 92 im Bereich der Mitte des Bildschirms 70 zusammen vergrößert darzustellen, wie dies in Figur 26 beispielhaft gezeigt wird. Hierbei ist es vorteilhaft, das oben beschriebene Prinzip der Tiefen- bzw. lateralen Navigation auch hier einzusetzen, wobei entsprechende Geraden 93 und 94 in die dargestellten Slice- bzw. En-face-Bilder 91 bzw. 92 eingeblendet werden. Durch die Auswahl einer Gerade 93 bzw. 94 sowie durch die Wahl der Lage der ausgewählten Gerade 93 bzw. 94 im Bereich des En-face-Bildes 92 kann der Benutzer die Ebene des jeweils angezeigten Slice-Bildes 91 festlegen. Darüber hinaus kann durch Auswahl und Verschiebung der Gerade 93 im Bereich des Slice-Bildes 91 eine Auswahl einer Ebene des aus dem dreidimensionalen Tomogramm stammenden En-face-Bildes, das angezeigt werden soll, erfolgen.

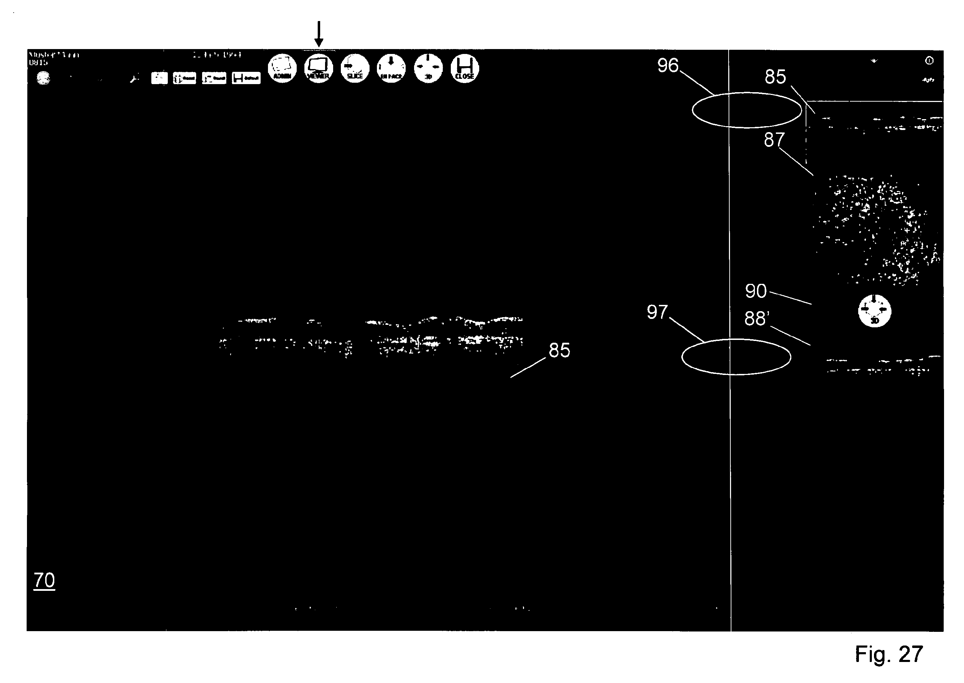

Anhand der in Figur 27 dargestellten Bildschirmansicht 70 wird veranschaulicht, wie im Bildbetrachtungsmodus Kommentare eingegeben werden können. Dazu wird vom Bediener zunächst ein zu kommentierendes OCT-Bild ausgewählt, im dargestellten Beispiel ist dies das Slice-Bild 85, und daraufhin ein entsprechendes, diesem Bild zugeordnetes, Kommentarfeld 97 geöffnet, in welches dann in Form von Freitext beliebige Kommentare eingegeben werden können. Darüber hinaus wird ein allgemeines Kommentarfeld 96 geöffnet, in welches ein Kommentar zur durchgeführten Untersuchung eingegeben werden kann, welcher der Gesamtheit der bei dieser Untersuchung aufgenommenen OCT-Bilder 85, 87, 88' und 90 zugeordnet wird und bei Abrufen zumindest eines dieser Bilder zusammen mit dem abgerufenen Bild angezeigt wird. In der Mitte des in Figur 27 gezeigten Bildschirms 70 wird das im rechten Bereich des Bildschirms 70 ausgewählte und verkleinert dargestellte Slice-Bild 85 in vergrößerter Form dargestellt.

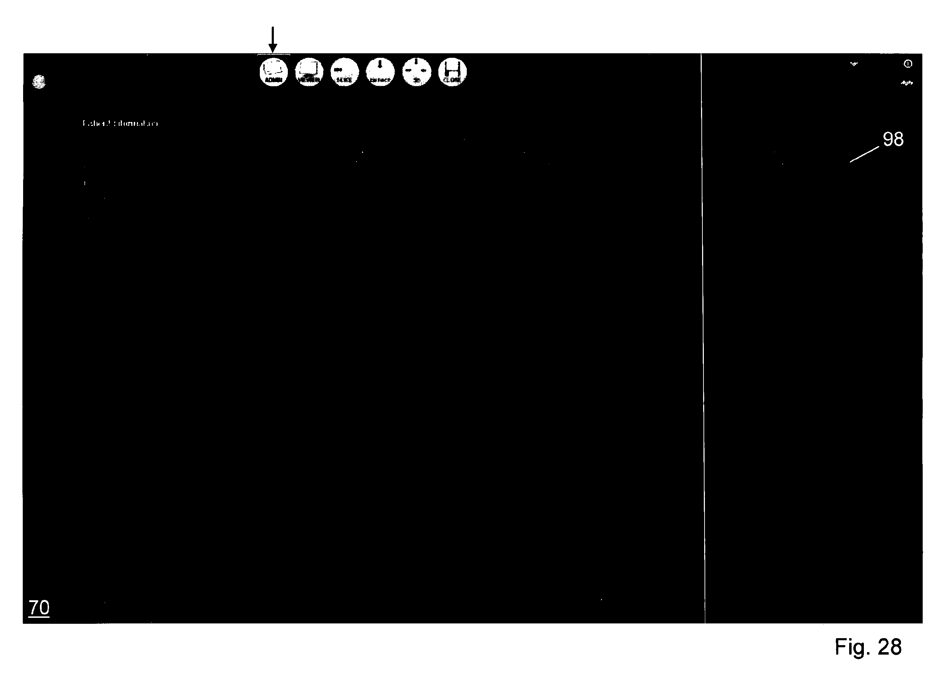

Nach Beendigung der Analyse und gegebenenfalls Kommentierung der bei der Untersuchung aufgenommenen OCT-Bilder kann ein Verwaltungsmodus des Systems 50 gewählt werden, in welchem in der Bildschirmansicht 70, wie in Figur 28 dargestellt, die durchgeführte Untersuchung in Form von jeweils einer Zeile 98 angezeigt wird. Durch Auswahl der entsprechenden Zeile 98 kann der Bediener erneut in den Bildbetrachtungsmodus wechseln und die aufgenommenen OCT-Bilder analysieren und gegebenenfalls kommentieren.

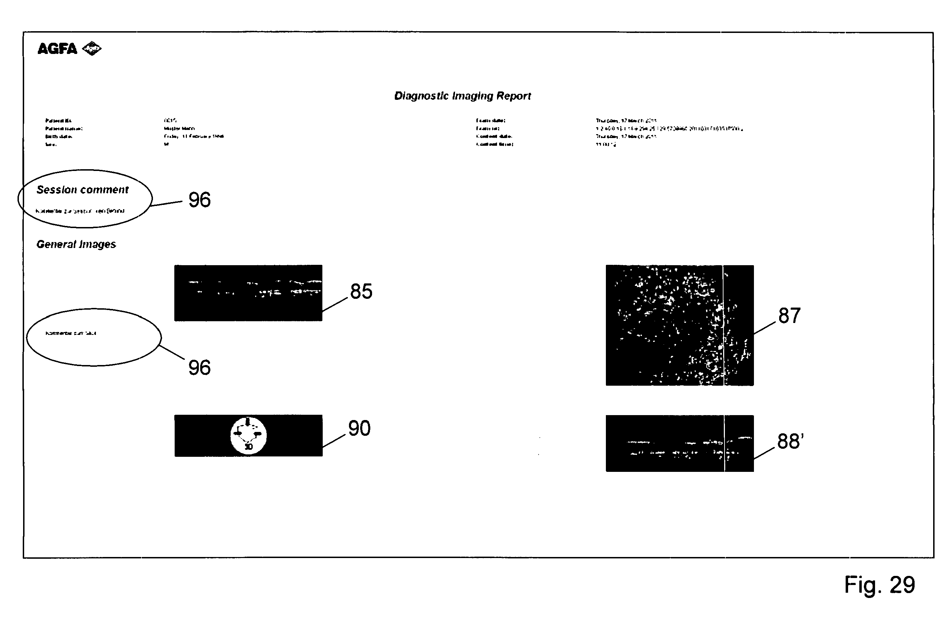

Von der durchgeführten Untersuchung kann - automatisch nach Beendigung der Untersuchung oder nach einem Benutzerbefehl - ein Untersuchungsbericht erstellt werden, wie er beispielhaft in Figur 29 gezeigt ist. Im Untersuchungsbericht, der vorzugsweise im HTML-Format erstellt wird, werden neben den vor der Untersuchung eingegebenen Patienteninformationen die bei der Untersuchung aufgenommenen und aufgrund eines Benutzerbefehls gespeicherten OCT-Bilder 85, 87, 88' und 90 sowie die jeweils eingegebenen Kommentare 96 und 97 in Form einer Übersicht zusammengestellt.

## Patentansprüche

1. Verfahren zur optischen Kohärenztomographie, bei welchem mittels einer optischen Kohärenztomographieeinrichtung (10 - 30) mindestens zwei zweidimensionale Ausgangsbilder (S; F) eines Objekts (1) in voneinander beabstandeten, parallel zueinander verlaufenden Ebenen des Objekts (1) aufgenommen werden, wobei die Ausgangsbilder (S; F) jeweils eine Vielzahl von Ausgangsbildwerten umfassen und eine Interpolation der Ausgangsbildwerte der mindestens zwei zweidimensionalen Ausgangsbilder (S; F) im dreidimensionalen Raum durchgeführt wird und dabei Interpolationswerte erhalten werden, die ein zweidimensionales Endbild (S'; F') bilden, wobei die Ausgangsbildwerte der mindestens zwei zweidimensionalen Ausgangsbilder (S; F) in einem regelmäßigen Gitter im dreidimensionalen Raum liegen,
**dadurch gekennzeichnet,**
**dass** benachbarte Ausgangsbildwerte in dem Gitter in allen drei Raumrichtungen gleiche Abstände zueinander aufweisen.

2. Verfahren nach Anspruch 1, wobei die gleichen Abstände in dem regelmäßigen Gitter zwischen etwa 2 pm und 4 µm liegen.

3. Verfahren nach Anspruch 1 oder 2, wobei die Ausgangsbildwerte der mindestens zwei zweidimensionalen Ausgangsbilder (S; F) durch eine trilineare Interpolation und/oder eine triquadratische und/oder eine trikubische Interpolation interpoliert werden.

4. Verfahren nach einem der vorangehenden Ansprüche, wobei die mindestens zwei zweidimensionalen Ausgangsbilder (S; F) Echtzeitbilder sind, welche mit einer Rate von mindestens einem Bild pro Sekunde, vorzugsweise mindestens fünf Bildern pro Sekunde, aufgenommen werden.

5. Verfahren nach einem der vorangehenden Ansprüche, wobei die mindestens zwei zweidimensionalen Ausgangsbilder (S) in voneinander beabstandeten Ebenen des Objekts (1) in einem ersten Betriebsmodus aufgenommen werden, bei welchem vom Objekt (1) reflektiertes bzw. zurückgestreutes Licht nur von einer Teilfläche (A2), insbesondere von zwei benachbarten Zeilen, eines ortsauflösenden Detektors (30) der optischen Kohärenztomographieeinrichtung (10 - 30) erfasst wird, während der optische Abstand eines Reflektors (16) zu einem Strahlteiler (11) der optischen Kohärenztomographieeinrichtung (10 - 30) um einen optischen Weg verändert wird, der wesentlich, insbesondere mindestens 100 mal, größer ist als die mittlere Wellenlänge (λ₀) von in die optische Kohärenztomographieeinrichtung (10 - 30) eingekoppeltem Licht.

6. Verfahren nach einem der vorangehenden Ansprüche, wobei die mindestens zwei zweidimensionalen Ausgangsbilder (F) des Objekts (1) in voneinander beabstandeten Ebenen des Objekts (1) in einem zweiten Betriebsmodus aufgenommen werden, bei welchem während einer Veränderung des optischen Abstandes eines Reflektors (16) zu einem Strahlteiler (11) der optischen Kohärenztomographieeinrichtung (10 - 30) das von dem Objekt (1) reflektierte Licht von Detektorelementen (31) eines Detektors (30) mehrmals, insbesondere höchstens fünfmal, erfasst wird, wobei die Veränderung des optischen Abstands des Reflektors (16) zum Strahlteiler (11) höchstens das Vierzigfache der mittleren Wellenlänge (λ₀) von in die optische Kohärenztomographieeinrichtung (10 - 30) eingekoppeltem Licht beträgt.

7. Verfahren nach Anspruch 6, wobei die beiden Ebenen in unterschiedlichen Tiefen (P₁, P₂) im Objekt (1) oberhalb bzw. unterhalb einer mittleren Tiefe (P₀) im Objekt (1) verlaufen.

8. Verfahren nach Anspruch 7, wobei die beiden Ebenen einen gleichen Abstand von der mittleren Tiefe (P₀) aufweisen.

9. Verfahren nach Anspruch 2 und einem der Ansprüche 7 oder 8, wobei der Abstand der beiden Ebenen zueinander dem in den drei Raumrichtungen gleichen Abstand der Ausgangsbildwerte entspricht.

10. Verfahren nach einem der Ansprüche 6 bis 9, wobei die mittlere Tiefe (P₀) bzw. die unterschiedlichen Tiefen (P₁, P₂) der oberhalb bzw. unterhalb der mittleren Tiefe (P₀) im Objekt (1) verlaufenden Ebenen durch den Abstand des Reflektors (16) vom Strahlteiler (11) eingestellt wird.

11. Verfahren nach Anspruch 10, wobei der optische Abstand des Reflektors (16) zum Strahlteiler (11) der optischen Kohärenztomographieeinrichtung (10 - 30) um einen optischen Weg verändert wird, der wesentlich, insbesondere mindestens 100 mal, größer ist als die mittlere Wellenlänge (λ₀) des in die optische Kohärenztomographieeinrichtung (10 - 30) eingekoppelten Lichts.

12. Verfahren nach einem der vorangehenden Ansprüche, wobei vor Durchführung der Interpolation die Anzahl ursprünglicher Ausgangsbildwerte (42) der mindestens zwei zweidimensionalen Ausgangsbilder (F) in zumindest einer Dimension, insbesondere in Richtung der Tiefe (T) des Objekts (1), reduziert wird, indem jeweils mindestens zwei, vorzugsweise mehr als zehn, ursprüngliche Ausgangsbildwerte (42) zu einem Ausgangsbildwert zusammengefasst werden.

13. Verfahren nach Anspruch 12, wobei es sich bei den ursprünglichen Ausgangsbildwerten (42) um Abtastwerte handelt, die durch sukzessives Abtasten eines aus unterschiedlichen Tiefen (T) innerhalb eines Tiefenbereichs des Objekts (1) erhaltenen Interferenzmusters (40) erhalten werden.

14. Verfahren nach Anspruch 12 oder 13, wobei diejenigen ursprünglichen Ausgangsbildwerte (42) zusammengefasst werden, welche von einem Bereich, insbesondere Tiefenbereich, des Objekts (1) erhalten werden, dessen Ausdehnung (45) in der mindestens einen Dimension der Auflösung (41), insbesondere der axialen Auflösung oder Tiefenauflösung, der optischen Kohärenztomographieeinrichtung (10 - 30) entspricht.

15. System zur optischen Kohärenztomographie mit einer optischen Kohärenztomographieeinrichtung (10 - 30), eingerichtet zur Aufnahme von mindestens zwei zweidimensionalen Ausgangsbildern (S; F) eines Objekts (1) in voneinander beabstandeten, parallel zueinander verlaufenden Ebenen des Objekts (1), wobei die Ausgangsbilder jeweils eine Vielzahl von Ausgangsbildwerten umfassen, eine Verarbeitungseinrichtung, eingerichtet zur Interpolation der Ausgangsbildwerte der mindestens zwei zweidimensionalen Ausgangsbilder (S; F) im dreidimensionalen Raum, wobei Interpolationswerte erhalten werden, die ein zweidimensionales Endbild (S'; F') bilden und wobei die Ausgangsbildwerte der mindestens zwei zweidimensionalen Ausgangsbilder (S; F) in einem regelmäßigen Gitter im dreidimensionalen Raum liegen,
**dadurch gekennzeichnet, dass**
benachbarte Ausgangsbildwerte in dem Gitter in allen drei Raumrichtungen gleiche Abstände aufweisen.

## Claims

1. Method for optical coherence tomography, in which, by means of an optical coherence tomography device (10 - 30), at least two two-dimensional initial images (S; F) of an object (1) are recorded in planes of the object (1) which are spaced apart from one another and run parallel to one another, wherein each of the initial images (S; F) comprises a plurality of initial image values and wherein an interpolation of the initial image values of the at least two two-dimensional initial images (S; F) is carried out in the three-dimensional space, whereby interpolation values are obtained that form a two-dimensional final image (S'; F'), whereby the initial image values of the at least two two-dimensional initial images (S; F) are arranged in a regular grid in the three-dimensional space,
**characterized in that** neighbouring initial image values in the grid are equidistant in all three spatial directions.

2. Method according to claim 1, wherein the equal distances in the regular grid are between about 2 µm and 4 µm.

3. Method according to claim 1 or 2, wherein the initial image values of the at least two two-dimensional initial images (S; F) are interpolated by a trilinear interpolation and/or a triquadratic interpolation and/or a tricubic interpolation.

4. Method according to any one of the preceding claims, wherein the at least two two-dimensional initial images (S; F) are real time images which are recorded at a rate of at least one image per second, preferably at least five images per second.

5. Method according to any one of the preceding claims, wherein the at least two two-dimensional initial images (S) are recorded in spaced apart planes of the object (1) in a first operating mode in which light reflected or backscattered by the object (1) is detected only by a partial surface (A2), in particular two neighbouring lines, of a spatially resolving detector (30) of the optical coherence tomography device (10 - 30), while the optical distance of a reflector (16) from a beam splitter (11) of the optical coherence tomography device (10 - 30) is changed by an optical path that is significantly larger, in particular at least 100 times, than the mean wavelength (λ₀) of light injected into the optical coherence tomography device (10 - 30).

6. Method according to any one of the preceding claims, wherein the at least two two-dimensional initial images (F) of the object (1) are recorded in spaced apart planes of the object (1) in a second operating mode in which during a change of the optical distance of a reflector (16) from a beam splitter (11) of the optical coherence tomography device (10 - 30) the light reflected from the object (1) is detected several times, in particular at most five times, by detector elements (31) of a detector (30), wherein the change of the optical distance of the reflector (16) from the beam splitter (11) is at most forty times the mean wavelength (λ₀) of light injected into the optical coherence tomography device (10 - 30).

7. Method according to claim 6, wherein both planes extend in different depths (P₁, P₂) in the object (1) respectively above and below a mean depth (P₀) in the object (1).

8. Method according to claim 7, wherein both planes are equidistant to the mean depth (P₀).

9. Method according to claim 2 and any of claims 7 or 8, wherein the distance between both planes corresponds to the equal distance of the initial image values in the three spatial directions.

10. Method according to any of the claims 6 to 9, wherein, respectively, the mean depth (P₀) and the different depths (P₁, P₂) of the planes extending, respectively, above and below the mean depth (P₀) in the object (1) is (are) adjusted via the distance of the reflector (16) from the beam splitter (11).

11. Method according to claim 10, wherein the optical distance of the reflector (16) from the beam splitter (11) of the optical coherence tomography device (10 - 30) is changed by an optical path that is significantly larger, in particular at least 100 times, than the mean wavelength (λ₀) of the light injected into the optical coherence tomography device (10 - 30).

12. Method according to any one of the preceding claims, wherein, before carrying out the interpolation, the number of original initial image values (42) of the at least two two-dimensional initial images (F) is reduced in at least one dimension, in particular in the direction of the depth (T) of the object (1), by combining each time at least two, preferably more than ten, original initial image values (42) to one initial image value.

13. Method according to claim 12, wherein the original initial image values (42) are sampling values that are obtained by successively sampling an interference pattern (40) obtained from different depths (T) within a depth range of the object (1).

14. Method according to claim 12 or 13, wherein those initial image values (42) are combined that are obtained from a range, in particular a depth range, of the object (1) the extent (45) of which in the at least one dimension corresponds to the resolution (41), in particular the axial resolution or depth resolution, of the optical coherence tomography device (10 - 30).

15. System for optical coherence tomography, comprising an optical coherence tomography device (10 - 30) adapted for recording at least two two-dimensional initial images (S; F) of an object (1) in planes of the object (1) which are spaced apart from one another and run parallel to one another, wherein each of the initial images (S; F) comprises a plurality of initial image values, said system comprising a processing device adapted for interpolating the initial image values of the at least two two-dimensional initial images (S; F) in the three-dimensional space, whereby interpolation values are obtained that form a two-dimensional final image (S'; F') and whereby the initial image values of the at least two two-dimensional initial images (S; F) are arranged in a regular grid in the three-dimensional space, **characterized in that** neighbouring initial image values in the grid are equidistant in all three spatial directions.

## Revendications

1. Procédé de tomographie en cohérence optique comprenant l'acquisition, au moyen d'un dispositif de tomographie en cohérence optique (10 - 30), d'au moins deux images initiales bidimensionnelles (S; F) d'un objet (1) dans des plans de l'objet (1) qui sont espacés l'un de l'autre et qui sont parallèles l'un à l'autre, chacune des images initiales (S; F) comprenant une multitude de valeurs d'image initiale et une interpolation des valeurs d'image initiale desdites au moins deux images initiales bidimensionnelles (S; F) étant effectuée dans l'espace tridimensionnel, permettant d'obtenir des valeurs d'interpolation qui forment une image finale bidimensionnelle (S'; F'), lesdites valeurs d'image initiale desdites au moins deux images initiales bidimensionnelles (S; F) étant situées dans une grille régulière dans l'espace tridimensionnel,
**caractérisé en ce que** des valeurs d'image initiale adjacentes dans la grille sont équidistantes dans les trois directions spatiales.

2. Procédé selon la revendication 1, **caractérisé en ce que** les distances égales dans la grille régulière sont comprises entre environ 2 µm et 4 µm.

3. Procédé selon la revendication 1 ou 2, **caractérisé en ce que** les valeurs d'image initiale desdites au moins deux images initiales bidimensionnelles (S; F) sont interpolées par une interpolation trilinéaire et/ou une interpolation triquadratique et/ou une interpolation tricubique.

4. Procédé selon l'une quelconque des revendications précédentes, **caractérisé en ce que** lesdites au moins deux images initiales bidimensionnelles (S; F) sont des images en temps réel acquises à une cadence minimale d'une image par seconde, de préférence au moins cinq images par seconde.

5. Procédé selon l'une quelconque des revendications précédentes, **caractérisé en ce que** lesdites au moins deux images initiales bidimensionnelles (S) sont acquises dans des plans espacés l'un de l'autre de l'objet (1) dans un premier mode de fonctionnement dans lequel la lumière réfléchie ou rétrodiffusée par l'objet (1) n'est détectée que par une surface partielle (A2), en particulier par deux lignes adjacentes, d'un détecteur à résolution spatiale (30) du dispositif de tomographie en cohérence optique (10 - 30), la distance optique d'un réflecteur (16) à un diviseur de faisceau (11) du dispositif de tomographie en cohérence optique (10 - 30) étant modifiée par le biais d'un chemin optique qui est substantiellement plus long, en particulier au moins 100 fois, que la longueur d'onde moyenne (λ₀) de la lumière couplée dans le dispositif de tomographie en cohérence optique (10 - 30).

6. Procédé selon l'une quelconque des revendications précédentes, **caractérisé en ce que** lesdites au moins deux images initiales bidimensionnelles (F) de l'objet (1) sont acquises dans des plans espacés l'un de l'autre de l'objet (1) dans un deuxième mode de fonctionnement dans lequel, lors d'une modification de la distance optique d'un réflecteur (16) à un diviseur de faisceau (11) du dispositif de tomographie en cohérence optique (10 - 30), la lumière réfléchie par l'objet (1) est détectée plusieurs fois, en particulier au plus cinq fois, par des éléments de détecteur (31) d'un détecteur (30), ladite modification de la distance optique du réflecteur (16) au diviseur de faisceau (11) étant au plus quarante fois plus grande que la longueur d'onde moyenne (λ₀) de la lumière couplée dans le dispositif de tomographie en cohérence optique (10 - 30).

7. Procédé selon la revendication 6, **caractérisé en ce que** les deux plans s'étendent dans des profondeurs différentes (P₁, P₂) dans l'objet (1) et ce respectivement au-dessus et en dessous d'une profondeur moyenne (P₀) dans l'objet (1).

8. Procédé selon la revendication 7, **caractérisé en ce que** les deux plans sont équidistants par rapport à la profondeur moyenne (P₀).

9. Procédé selon la revendication 2 et selon l'une quelconque des revendications 7 ou 8, **caractérisé en ce que** la distance entre les deux plans correspond à la distance égale des valeurs d'image initiale dans les trois directions spatiales.

10. Procédé selon l'une quelconque des revendications 6 à 9, **caractérisé en ce que** respectivement la profondeur moyenne (P₀) et les différentes profondeurs (P₁, P₂) des plans qui s'étendent respectivement au-dessus et en dessous de la profondeur moyenne (P₀) dans l'objet (1) est (sont) réglée(s) par le biais de la distance du réflecteur (16) au diviseur de faisceau (11).

11. Procédé selon la revendication 10, **caractérisé en ce que** la distance optique du réflecteur (16) au diviseur de faisceau (11) du dispositif de tomographie en cohérence optique (10 - 30) est modifiée par le biais d'un chemin optique qui est substantiellement plus long, en particulier au moins 100 fois, que la longueur d'onde moyenne (λ₀) de la lumière couplée dans le dispositif de tomographie en cohérence optique (10 - 30).

12. Procédé selon l'une quelconque des revendications précédentes, **caractérisé en ce que**, avant d'effectuer l'interpolation, le nombre de valeurs d'image initiale originale (42) desdites au moins deux images initiales bidimensionnelles (F) est réduit dans au moins une dimension, en particulier dans la direction de la profondeur (T) de l'objet (1), en résumant chaque fois au moins deux, de préférence plus de dix, valeurs d'image initiale originale (42) en une valeur d'image initiale.

13. Procédé selon la revendication 12, **caractérisé en ce que** les valeurs d'image initiale originales (42) sont des valeurs d'échantillonnage obtenues en échantillonnant successivement un motif d'interférence (40) obtenu à partir de différentes profondeurs (T) dans une plage de profondeurs de l'objet (1).

14. Procédé selon la revendication 12 ou 13, **caractérisé en ce que** sont résumées les valeurs d'image initiale (42) qui sont obtenues à partir d'une plage, en particulier une plage de profondeurs, de l'objet (1) dont l'étendue (45) dans ladite au moins une dimension correspond à la résolution (41), en particulier la résolution axiale ou la résolution de profondeur, du dispositif de tomographie en cohérence optique (10 - 30).

15. System de tomographie en cohérence optique comprenant un dispositif de tomographie en cohérence optique (10 - 30) adapté de façon à assurer l'acquisition d'au moins deux images initiales bidimensionnelles (S; F) d'un objet (1) dans des plans de l'objet (1) qui sont espacés l'un de l'autre et qui sont parallèles l'un à l'autre, chacune des images initiales (S; F) comprenant une multitude de valeurs d'image initiale, ledit système comprenant un dispositif de traitement adapté de façon à assurer l'interpolation des valeurs d'image initiale desdites au moins deux images initiales bidimensionnelles (S; F) dans l'espace tridimensionnel, permettant d'obtenir des valeurs d'interpolation qui forment une image finale bidimensionnelle (S'; F'), lesdites valeurs d'image initiale desdites au moins deux images initiales bidimensionnelles (S; F) étant situées dans une grille régulière dans l'espace tridimensionnel,
**caractérisé en ce que** des valeurs d'image initiale adjacentes dans la grille sont équidistantes dans les trois directions spatiales.
